# EUROPÄISCHE PATENTSCHRIFT

(11) **EP 1 013 652 B1**
(45) Veröffentlichungstag und Bekanntmachung des Hinweises auf die Patenterteilung: **14.08.2002**
(21) Anmeldenummer: 99124191.0
(22) Anmeldetag: 03.12.1999
(51) Int. Cl.: C07D 311/70, C07D 311/66, C07D 311/74, C07D 309/30, C07D 407/04

(54) **Verfahren zur Herstellung von substituierten Chromanderivaten**
Process for preparation of substituted chromanderivatives
Procédé de préparation de dérivés de chromane substitués

(30) Priorität: 22.12.1998 DE 19859251
(43) Veröffentlichungstag der Anmeldung: 28.06.2000
(73) Patentinhaber: BASF AKTIENGESELLSCHAFT, 67056 Ludwigshafen (DE)
(72) Erfinder: Stürmer, Rainer, Dr., 67127 Rödersheim-Gronau (DE); Baldenius, Kai-Uwe, Dr., 67227 Frankenthal (DE)

(56) Entgegenhaltungen:
- CURTIS,W. SMITH ET AL.: "REACTIONS OF ACROLEIN AND RELATED COMPOUNDS.I.ADDITION OF VINYL ETHERS." JOURNAL OF THE AMERICAN CHEMICAL SOCIETY., Bd. 73, November 1951 (1951-11), Seiten 5267-70, XP002094064 AMERICAN CHEMICAL SOCIETY, WASHINGTON, DC., US ISSN: 0002-7863
- TSUENO SATO ET AL.: "ORGANOTIN TRIFLATES AS FUNCTIONAL LEWIS ACIDS." TETRAHEDRON LETTERS., Bd. 31, Nr. 11, 1990, Seiten 1581-4, XP002145348 ELSEVIER SCIENCE PUBLISHERS, AMSTERDAM., NL ISSN: 0040-4039

## Beschreibung

Die Erfindung betrifft ein Verfahren zur Herstellung substituierter Chromanderivate der allgemeinen Formel VII, 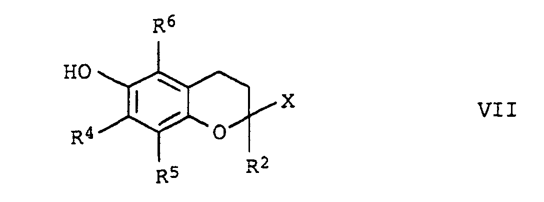 wobei
- X: die Gruppe -CN, -COOR³, -CHO, -CH₂OR⁷ oder -CH(OR⁸)₂,
- R²: einen C₁-C₂₃-Alkyl-, C₂-C₂₃-Alkenyl, C₆-C₁₈-Aryl-, oder C₇-C₁₈-Aralkyl-Rest,
- R³: Wasserstoff, einen C₁-C₄-Alkylrest, einen C₁-C₄-Halogenalkylrest, einen C₁-C₄-Hydroxyalkylrest oder einen C₁-C₄-Aminoalkylrest,
- R⁴, R⁵, R⁶: unabhängig voneinander Wasserstoff oder einen C₁-C₄-Alkylrest,
- R⁷: Wasserstoff oder einen C₁-C₄-Alkylrest,
- R⁸: einen C₁-C₄-Alkylrest, oder beide Reste einen C₂-C₆-Alkylenrest bedeuten, der die beiden Sauerstoffatome im Sinne eines cyclischen Acetals verbrückt und gegebenenfalls verzweigt ist oder ein bis zwei Carboxylgruppen, Cyclohexyl- oder Phenylreste tragen kann,
darstellen, sowie die neuen Zwischenprodukte des Verfahrens.

Die substituierten Chromanderivate der allgemeinen Formel VII sind gesuchte, bekannte Zwischenprodukte für pharmazeutische Wirkstoffe und Vitamine (z.B. Tocopherol). Ausgehend von 6-Acetyloxy-chroman-2-aldehyd konnte unter anderem α-Tocopherol durch Wittig-Reaktion mit einem Phosphoniumsalz hergestellt werden (T. Netscher, Chimia 1996, 50, 563-567; H. Mayer, P. Schudel, R. Ruegg, O. Isler, Helv. Chim. Acta 1963, 46, 650). Darüber hinaus zeigen diese Verbindungen, wie z.B. die 6-Hydroxychroman-2-carboxylsäuren in tierischem Fett, pflanzlichen Ölen und Emulsionen selbst eine antioxidierende Wirkung (J. W. Scott et al., J. of the American Oil Chemists Society, 1974, 51, 200-203).
Es ist bekannt, die substituierten Chromanderivate über die Kondensation der entsprechenden Hydrochinone mit Allylalkoholen herzustellen (US 2411969, WO 9821197 A2).
Ferner beschreibt DE 2364141 A1 einen ähnlichen Zugang durch Umsetzung eines Hydrochinons mit ungesättigten Ketonen oder Acetalen in Gegenwart einer Säure.
Gemeinsam ist allen bisher beschriebenen Verfahren, daß der Aromat als Synthesebaustein eingesetzt und der Pyranteil anschließend durch elektrophile aromatische Substitutionen aufgebaut wird.
Diese Reaktionen haben den Nachteil, daß am Aromaten unsubstituierte Chromane schlecht herzustellen sind, da diese elektronenreichen Aromaten zur Mehrfachalkylierung neigen.
Auch mit unterschiedlichen Alkylgruppen am Aromaten treten bei den oben genannten Synthesen Regioselektivitätsprobleme auf, d.h. es lassen sich nicht alle gewünschten Substitutionsmuster am aromatischen Teil des Chromangerüstes gleich gut herstellen.

Es stellte sich daher die Aufgabe, ein in Bezug auf die Substituenten flexibleres, wirtschaftliches und über leicht zu handhabende Zwischenprodukte und Reagenzien ablaufendes Verfahren zur Herstellung der Chromanderivate zu entwickeln.

Demgemäß wurde gefunden daß man die eingangs erwähnten Chromanderivate der allgemeinen Formel VII durch ein mehrstufiges Verfahren herstellen kann, das dadurch gekennzeichnet ist, daß man in einem ersten Schritt substituierte Acroleine der allgemeinen 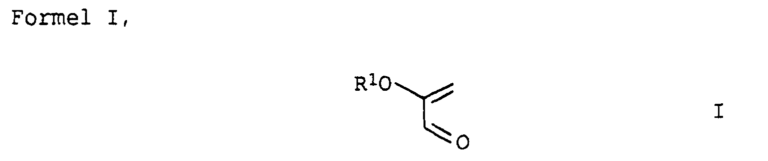 wobei
- R¹: einen C₁-C₄-Alkyl-, C₆-C₁₈-Aryl-, C₇-C₁₈-Aralkyl-, C₁-C₄-Acyl-, einen halogenierten C₁-C₄-Acylrest oder eine andere säurelabile Schutzgruppe der Hydroxyfunktion darstellt,
mit Acrylnitrilen, Acrylaten, Acroleinen, Acroleinacetalen, Allylalkoholen oder Allylethern der allgemeinen Formel II 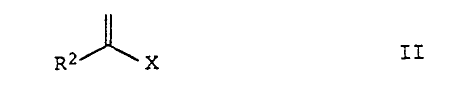 zu den entsprechenden 3,4-Dihydro-2H-Pyranen der allgemeinen Formel III 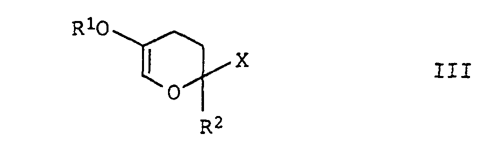 umsetzt und diese in einem zweiten Schritt mit einer Säure zu den entsprechenden 5-Oxo-tetrahydropyranen der allgemeinen Formel IV 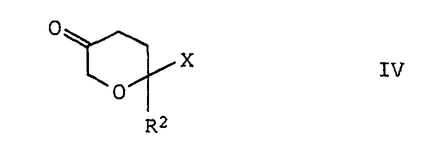 umsetzt und diese in einem dritten Schritt mit substituierten Vinylketonen der allgemeinen Formel V 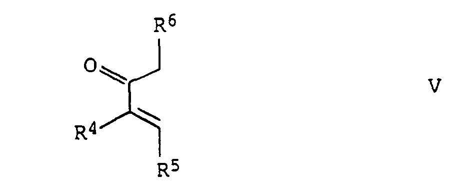 zu den entsprechenden Chromen-6-on-Derivaten der allgemeinen Formel VI 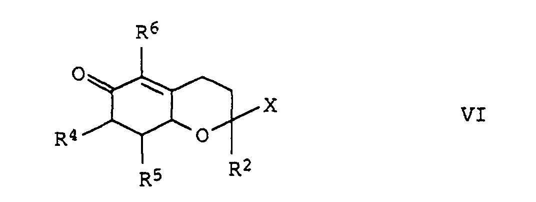 umsetzt und diese in einem 4 Schritt zu den substituierten Chromanderivaten der allgemeinen Formel VII dehydriert.

Ausgangsstoffe des Verfahrens sind substituierte Acroleine der allgemeinen Formel I und Acrylnitrile, Acrylate, Acroleine, Acroleinacetale, Allylalkohole oder Allylether der allgemeinen Formel II.

Die substituierten Acroleine der allgemeinen Formel I stellen im ersten Schritt des erfindungsgemäßen Verfahrens die Dien-Komponente einer Hetero-Diels-Alder-Reaktion dar.

Der Rest R¹ der substituierten Acroleine kann einen C₁-C₄-Alkyl-, C₆-C₁₈-Aryl-, C₇-C₁₈-Aralkyl-, einen gegebenenfalls halogenierten C₁-C₄-Acylrest oder eine andere säurelabile Schutzgruppe der Hydroxyfunktion bedeuten. Unter C₁-C₄-Alkylrest wird ein Methyl-, Ethyl-, n-Propyl-, i-Propyl, n-Butyl-, i-Butyl-, oder t-Butyl-Rest verstanden, wobei Methyl bevorzugt ist. C₆-C₁₈-Arylreste sind beispielsweise Phenyl, Naphthyl oder gegebenenfalls ein bis dreifach mit C₁-C₄-Alkylresten substituiertes Phenyl. Unter C₇-C₁₈-Aralkylresten werden Reste wie beispielsweise Benzyl oder Phenylethyl verstanden.
C₁-C₄-Acylreste oder halogenierte, d.h. mit ein bis drei gleichen oder verschiedenen Halogenresten wie Cl, Br, I oder F substituierte, C₁-C₄-Acylreste sind beispielsweise Acetyl, Monochlor-, Dichlor- oder Trichloracetyl, Trifluoracetyl, Propionyl oder Butyryl.
R¹ kann auch eine andere säurelabile Schutzgruppe der Hydroxyfunktion des Enols darstellen. Prinzipiell kann jede säurelabile Schutzgruppe verwendet werden. Bevorzugte säurelabile Schutzgruppen sind die in der Literatur bekannten säurelabilen Schutzgruppen für Hydroxygruppen (T.W. Greene, Protective Groups in Organic Synthesis, John Wiley & Sons New York, 1981,Seite 14-71; P.J. Kocienski, Protecting Groups, Georg Thieme Verlag Stuttgart, 1994, Seite 21-94). Besonders bevorzugte säurelabile Schutzgruppen sind

Ester, wie
Phenylacetate, Triphenylmethoxyacetat, Phenoxyacetate, Halogenphenoxyacetate, Halogenalkylphenoxyacetate, Formiat, Benzoylformiat, 3-Phenylpropionat, Isobutyrat, Pivaloat, Adamantoat, Crotonate oder Benzoate.
oder
aliphatische und aromatische Ether, wie
Methyl , Benzyl , o-Nitrobenzyl, p-Methoxybenzyl, 3,4-Dimethoxybenzylether, Trityle, p-Methoxyphenyl-diphenylmethyl, 4, 4', 4''-tris(benzoyloxy)trityl , di(p-Methoxyphenyl)phenylmethyl, tert-Butyle, 9-Phenyl-9-xanthenyl, Allyle, 2-(Trimethylsilyl)-ethyl

Bevorzugte substituierte Acroleine der allgemeinen Formel I sind α-Methoxyacrolein, α-Benzyloxyacrolein oder Acetoxyacrolein.

Die substituierten Acrylnitrile (X = -CN), Acroleine (X = -CHO), Acrylate (X = -COOR³), Acroleinacetale (x = -CH(OR⁸)₂), Allylether (X = -CH₂OR⁷) oder Allylalkohole (X = -CH₂OR⁷; R⁷ = Wasserstoff) der allgemeinen Formel II stellen im ersten Schritt des erfindungsgemäßen Verfahrens die En-Komponente einer Hetero-Diels-Alder-Reaktion dar.

Der Rest R² kann einen C₁-C₂₃-Alkylrest, wie Methyl, Ethyl, n-Propyl, i-Propyl, n-Butyl, i-Butyl, t-Butyl, Pentyl, Hexyl, Heptyl, Octyl, Nonyl oder 4,8,12-Trimethyl-tridecyl, insbesondere Methyl, einen C₂-C₂₃-Alkenylrest, wie Ethenyl(Vinyl), Propenyl (Allyl), 4-Methyl-penta-3-enyl, 4,8-Dimethyl-nona-3,7-dienyl oder 4,8,12-Trimethyl-trideka-3,7,11-trienyl,
einen C₆-C₁₈-Arylrest wie vorstehend für R¹ erwähnt, insbesondere Phenyl,
oder einen C₇-C₁₈-Aralkylrest, wie vorstehend für R¹ erwähnt, insbesondere Benzyl bedeuten.

Bevorzugte mit R² substituierte Acrylnitrile (X = -CN) der Formel II sind 2-Methyl-acrylnitril, 2-(4,8,12-Trimethyltridecyl)-acrylnitril, 2-(4-Methyl-penta-3-enyl)-acrylnitril, 2-(4,8-Dimethyl-nona-3,7-dienyl)-acrylnitril oder 2-(4,8,12-Trimethyl-trideka-3,7,11-trienyl)-acrylnitril.

Bevorzugte mit R² substituierte Acroleine (X = -CHO) der Formel II sind 2-Methylacrolein (Methacrolein), 2-(4,8,12-Trimethyl-tridecyl)-acrolein, 2-(4-Methyl-penta-3-enyl)-acrolein, 2-(4,8-Dimethyl-nona-3,7-dienyl)-acrolein oder 2-(4,8,12-Trimethyl-trideka-3,7,11-trienyl)-acrolein.

Unter mit R² substituierten Acrylaten (X = -COOR³) werden substituierte Acrylsäure (R³ = Wasserstoff) oder Acrylsäureester (R³ = ein C₁-C₄-Alkylrest, ein C₁-C₄-Halogenalkylrest, ein C₁-C₄-Hydroxyalkylrest oder ein C₁-C₄-Aminoalkylrest) verstanden. Unter C₁-C₄-Alkylresten für R³ werden die, wie vorstehend für R¹ erwähnten C₁-C₄-Alkylreste, insbesondere Methyl oder Ethyl verstanden.
Unter C₁-C₄-Halogenalkylresten werden C₁-C₄-Alkylreste verstanden, die mit einem bis drei gleichen oder verschiedenen Halogenresten, wie F, Cl, Br, oder I substituiert sind. C₁-C₄-Halogenalkylreste sind beispielsweise 2-Chlorethyl, 1,2-Dichlorethyl oder 2,2,2-Trichlorethyl und die entsprechenden Bromethylreste. Unter C₁-C₄-Hydroxyalkylresten werden C₁-C₄-Alkylreste verstanden, die mit einer bis drei Hydroxygruppen substituiert sind. C₁-C₄-Hydroxyalkylreste sind beispielsweise 2-Hydroxyethyl oder 1,2-Dihydroxyethyl.
Unter C₁-C₄-Aminoalkylresten, werden C₁-C₄-Alkylresten verstanden, die mit einer bis drei Aminogruppen substituiert sind. Unter Aminogruppen werden NH₂-Gruppen, Mono(C₁-C₄-)alkylaminogruppen oder Di(C₁-C₄-)alkylaminogruppen verstanden. C₁-C₄-Aminoalkylreste sind beispielsweise Aminoethyl, 2-Methylaminoethyl, 2-Dimethylaminoethyl oder Ethylmethylaminoethyl.

Bevorzugte, substituierte Acrylate der allgemeinen Formel II sind 2-Methylacrylsäure (Methacrylsäure), Methacrylsäure-methylester, Methacrylsäure-ethylester, Methacrylsäure-(2-Hydroxyethyl)-ester, Methacrylsäure-(2-chlorethyl)-ester, Methacrylsäure-(2-dimethylaminoethyl)-ester, 2-(4,8,12-Trimethyl-tridecyl)-acrylsäure, 2-(4-Methyl-penta-3-enyl)-acrylsäure, 2-(4,8-Dimethyl-nona-3,7-dienyl)-acrylsäure, 2-(4,8,12-Trimethyl-trideka-3,7,11-trienyl)-acrylsäure, 2-(4,8,12-Trimethyl-tridecyl)-acrylsäuremethylester, 2-(4-Methyl-penta-3-enyl)-acrylsäure-methylester, 2-(4,8-Dimethyl-nona-3,7-dienyl)-acrylsäure-methylester, 2-(4,8,12-Trimethyl-trideka-3,7,11-trienyl)-acrylsäure-methylester, 2-(4,8,12-Trimethyl-tridecyl)-acrylsäure-ethylester, 2-(4-Methyl-penta-3-enyl)-acrylsäure-ethylester, 2-(4,8-Dimethylnona-3,7-dienyl)-acrylsäure-ethylester, 2-(4,8,12-Trimethyl-trideka-3,7,11-trienyl)- acrylsäure-ethylester, 2-(4,8,12-Trimethyltridecyl)-acrylsäure-(2-Hydroxyethyl)-ester, 2-(4-Methyl-penta-3-enyl)-acrylsäure-(2-Hydroxyethyl)-ester, 2-(4,8-Dimethyl-nona-3, 7-dienyl)-acrylsäure-(2-Hydroxyethyl)-ester, 2-(4,8,12-Trimethyl-trideka-3,7, 11-trienyl)- acrylsäure-(2-Hydroxyethyl)-ester, 2-(4,8,12-Trimethyl-tridecyl)-acrylsäure-(2-Chlorethyl)-ester, 2-(4-Methyl-penta-3-enyl)-acrylsäure-(2-Chlorethyl)-ester, 2-(4,8-Dimethyl-nona-3,7-dienyl)-acrylsäure-(2-Chlorethyl)-ester oder 2-(4,8,12-Trimethyl-trideka-3,7,11-trienyl)-acrylsäure-(2-Chlorethyl)-ester, 2-(4,8,12-Trimethyl-tridecyl)-acrylsäure-(2-dimethylaminoethyl)-ester, 2-(4-Methyl-penta-3-enyl)-acrylsäure-(2-dimethylaminoethyl)-ester, 2-(4,8-Dimethyl-nona-3,7-dienyl)-acrylsäure-(2-dimethylaminoethyl)-ester oder 2-(4,8,12-Trimethyl-trideka-3,7,11-trienyl)-acrylsäure-(2-dimethylaminoethyl)-ester.

Im Fall der mit R² substituierten Allylether (X = -CH₂OR⁷) oder Allylalkohole (X = -CH₂OR⁷; R⁷ = Wasserstoff) kann R⁷ Wasserstoff oder einen C₁-C₄-Alkylrest, wie vorstehend für R¹ beschrieben, insbesondere Methyl oder Ethyl bedeuten.
Bevorzugte, substituierte Allylether der allgemeinen Formel II sind 2-Methyl-3-Methoxypropen, 2-Methyl-3-Ethoxypropen, 2-(4,8,12-Trimethyl-tridecyl)-3-Methoxypropen, 2-(4-Methylpenta-3-enyl)-3-Methoxypropen, 2-(4,8-Dimethyl-nona-3,7-dienyl)-3-Methoxypropen, 2-(4,8,12-Trimethyl-trideka-3,7,11-trienyl)-3-Methoxypropen, 2-(4,8,12-Trimethyl-tridecyl)-3-Ethoxypropen, 2-(4-Methyl-penta-3-enyl)-3-Ethoxypropen, 2-(4,8-Dimethyl-nona-3,7-dienyl)-3-Ethoxypropen oder 2-(4,8,12-Trimethyl-trideka-3,7,11-trienyl)-3-Ethoxypropen.

Bevorzugte, substituierte Allylalkohole der allgemeinen Formel II sind 2-Methyl-prop-2-en-1-ol (2-Methyl-Allylalkohol, Methallyl-alkohol), 2-(4,8,12-Trimethyl-tridecyl)-prop-2-en-1-ol, 2-(4-Methyl-penta-3-enyl)-prop-2-en-1-ol, 2-(4,8-Dimethylnona-3,7-dienyl)-prop-2-en-1-ol oder 2-(4,8,12-Trimethyltrideka-3,7,11-trienyl)-prop-2-en-1-ol.

Im Fall der mit R² substituierten Acroleinacetale (X = -CH(OR⁸)₂) bedeutet R⁸ einen C₁-C₄-Alkylrest, wie vorstehend für R¹ beschrieben, insbesondere Methyl, oder beide Reste R⁸ einen, die beiden Sauerstoffatome im Sinne eines cyclischen Acetals verbrückenden, gegebenenfalls verzweigten oder bis zu zwei Carboxylgruppen, Cyclohexyl- oder Phenylreste tragenden, C₂-C₆-Alkylen-Rest. Bevorzugte C₂-C₆-Alkylen-Reste sind Ethylen, Propylen, Butylen oder Neopentylen. Bevorzugte, bis zu zwei Carboxylgruppen, Cyclohexyloder Phenylreste tragende C₂-C₆-Alkylen-Reste sind Ethylenreste, die in 1- und/oder 2-Position durch die genannten Reste substituiert sind. Bevorzugt sind symmetrisch substituierte C₂-Brücken wie im folgenden aufgezählt, wobei die Verknüpfungsstellen durch einen, die Stereochemie angebenden, Bindungsstrich gekennzeichnet sind:

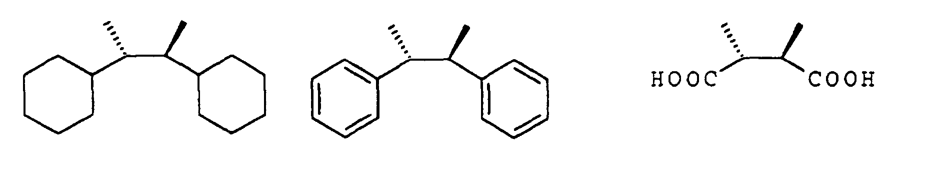

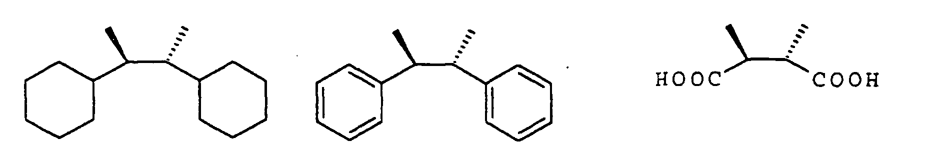

Bevorzugte, substituierte Acroleinacetale der allgemeinen Formel II sind dementsprechend 2-Methyl-3,3-dimethoxy-propen, 2-Methyl-3,3-diethoxy-propen, 2-Methyl-3,3-dipropoxy-propen, 2-Methyl-3,3-dibutoxy-propen, 2-(4,8,12-Trimethyl-tridecyl)-3,3-dimethoxy-propen, 2-(4,8,12-Trimethyl-tridecyl)-3,3-diethoxypropen, 2-(4,8,12-Trimethyl-tridecyl)-3,3-dipropoxy-propen, 2-(4,8,12-Trimethyl-tridecyl)-3,3-dibutoxy-propen, 2-(4,8,12-Trimethyl-tridecyl)-3,3-dimethoxy-propen, 2-(4,8,12-Trimethyl-tridecyl)-3,3-diethoxy-propen, 2-(4,8,12-Trimethyl-tridecyl)-3,3-dipropoxy-propen, 2-(4,8,12-Trimethyl-tridecyl)-3,3-dibutoxypropen, 2-(4-Methyl-penta-3-enyl)-3,3-dimethoxy-propen, 2- (4-Methyl-penta-3-enyl)-3,3-diethoxy-propen, 2-(4-Methylpenta-3-enyl)-3,3-dipropoxy-propen, 2-(4-Methyl-penta-3-enyl)-3,3-dibutoxy-propen, 2-(4,8-Dimethyl-nona-3,7-dienyl)-3,3-dimethoxy-propen, 2-(4,8-Dimethyl-nona-3,7-dienyl)-3,3-diethoxypropen, 2-(4,8-Dimethyl-nona-3,7-dienyl)-3,3-dipropoxy-propen, 2-(4,8-Dimethyl-nona-3,7-dienyl)-3,3-dibutoxy-propen, 2-(4,8,12-Trimethyl-trideka-3,7,11-trienyl)-3,3-dimethoxy-propen, 2-(4,8,12-Trimethyl-trideka-3,7,11-trienyl)-3,3-diethoxy-propen, 2-(4,8,12-Trimethyl-trideka-3,7,11-trienyl)-3,3-dipropoxy-propen, 2-(4,8,12-Trimethyl-trideka-3,7,11-trienyl)-3,3-dibutoxy-propen, 2-(1-Methyl-vinyl)-4,5-dimethyl[1,3]dioxan, 2-(1-(4,8,12-Trimethyl-tridecyl)-vinyl)-4,5-dimethyl[1,3]dioxan, 2-(1-(4-Methylpenta-3-enyl)-vinyl)-4,5-dimethyl[1,3]dioxan, 2-(1-(4,8-Dimethyl-nona-3,7-dienyl)-vinyl)-4,5-dimethyl[1,3]dioxan, 2-(1-(4,8,12-Trimethyl-trideka-3,7,11-trienyl)-vinyl)-4,5-dimethyl[1,3]dioxan, 2-(1-Methyl-vinyl)-4,5-dicyclohexyl-[1,3]dioxolan, 2-(1-(4,8,12-Trimethyl-tridecyl)-vinyl)-4,5-dicyclohexyl-[1,3]dioxolan, 2-(1-(4-Methyl-penta-3-enyl)-vinyl)-4,5-dicyclohexyl-[1,3]dioxolan, 2-(1-(4,8-Dimethyl-nona-3,7-dienyl)-vinyl)-4,5-dicyclohexyl-[1,3]dioxolan, 2-(1-(4,8,12-Trimethyl-trideka-3,7,11-trienyl)-vinyl)-4,5-dicyclohexyl-[1,3]dioxolan, 2-(1-Methyl-vinyl)-[4R,5R]-4,5-dicyclohexyl-[1,3]dioxolan, 2-(1-(4,8,12-Trimethyl-tridecyl)-vinyl)-[4R,5R]-4,5-dicyclohexyl-[1,3]dioxolan, 2-(1-(4-Methyl-penta-3-enyl)-vinyl)-[4R,5R]-4,5-dicyclohexyl-[1,3]dioxolan, 2-(1-(4,8-Dimethyl-nona-3,7-dienyl)-vinyl)-[4R,5R]-4,5-dicyclohexyl-[1,3]dioxolan, 2-(1-(4,8,12-Trimethyl-trideka-3,7,11-trienyl)-vinyl)-[4R,5R]-4,5-dicyclohexyl-[1,3]dioxolan, 2-(1-Methylvinyl)-[4S,5S]-4,5-dicyclohexyl-[1,3]dioxolan, 2-(1-(4,8,12-Trimethyl-tridecyl)-vinyl)-[4S,5S]-4,5-dicyclohexyl-[1,3]dioxolan, 2-(1-(4-Methyl-penta-3-enyl)-vinyl)-[4S,5S]-4,5-dicyclohexyl-[1,3]dioxolan, 2-(1-(4,8-Dimethyl-nona-3,7-dienyl)-vinyl)-4,5-dicyclohexyl-[1,3]dioxolan, 2-(1-(4,8,12-Trimethyl-trideka-3,7,11-trienyl)-vinyl)-[4S,5S]-4,5-dicyclohexyl-[1,3]dioxolan,
2-(1-Methyl-vinyl)-4,5-diphenyl-[1,3]dioxolan, 2-(1-(4,8,12-Trimethyl-tridecyl)-vinyl)-4,5-diphenyl-[1,3]dioxolan, 2-(1-(4-Methyl-penta-3-enyl)-vinyl)-4,5-diphenyl-[1,3]dioxolan, 2-(1-(4,8-Dimethyl-nona-3,7-dienyl)-vinyl)-4,5-diphenyl-[1,3]dioxolan, 2-(1-(4,8,12-Trimethyl-trideka-3,7,11-trienyl)-vinyl)-4,5-diphenyl-[1,3]dioxolan, 2-(1-Methyl-vinyl)-[4R,5R]-4,5-diphenyl-[1,3]dioxolan, 2-(1-(4,8,12-Trimethyl-tridecyl)-vinyl)-[4R,5R]-4,5-diphenyl-[1,3]dioxolan, 2-(1-(4-Methylpenta-3-enyl)-vinyl)-[4R,5R]-4,5-diphenyl-[1,3]dioxolan, 2-(1-(4,8-Dimethyl-nona-3,7-dienyl)-vinyl)-[4R,5R]-4,5-diphenyl-[1,3]dioxolan, 2-(1-(4,8,12-Trimethyl-trideka-3,7,11-trienyl)-vinyl)-[4R,5R]-4,5-diphenyl-[1,3]dioxolan, 2-(1-Methyl-vinyl)-[4S,5S]-4,5-diphenyl-[1,3]dioxolan, 2-(1-(4,8, 12-Trimethyl-tridecyl)-vinyl)-[4S,5S]-4,5-diphenyl-[1,3]dioxolan, 2-(1-(4-Methyl-penta-3-enyl)-vinyl)-[4S,5S]-4,5-diphenyl-[1,3]dioxolan, 2-(1-(4,8-Dimethyl-nona-3,7-dienyl)-vinyl)-4,5-diphenyl-[1,3]dioxolan, 2-(1-(4,8,12-Trimethyl-trideka-3,7,11-trienyl)-vinyl)-[4S,5S]-4,5-diphenyl-[1,3]dioxolan, 2-(1-Methyl-vinyl)-[1,3]-dioxolan-4,5-dicarbonsäure, 2-(1-(4,8,12-Trimethyl-tridecyl)-vinyl)-[1,3]-dioxolan-4, 5-dicarbonsäure, 2-(1-(4-Methyl-penta-3-enyl)-vinyl)-[1,3]-dioxolan-4, 5-dicarbonsäure, 2-(1-(4, 8-Dimethyl-nona-3, 7-dienyl)-vinyl)-[1,3]-dioxolan-4,5-dicarbonsäure, 2-(1-(4,8,12-Trimethyltrideka-3,7,11-trienyl)-vinyl)-[1,3]-dioxolan-4, 5-dicarbonsäure, 2-(1-Methyl-vinyl)-[1,3]-dioxolan-[4R,5R]-4, 5-dicarbonsäure, 2-(1-(4,8,12-Trimethyl-tridecyl)-vinyl)-[1,3]-dioxolan-[4R,5R]-4, 5-dicarbonsäure, 2-(1-(4-Methyl-penta-3-enyl)-vinyl)-[1,3]-dioxolan-[4R,5R]-4,5-dicarbonsäure, 2-(1-(4,8-Dimethyl-nona-3,7-dienyl)-vinyl)-[1,3]-dioxolan-[4R,5R]-4,5-dicarbonsäure, 2-(1-(4,8,12-Trimethyl-trideka-3,7,11-trienyl)-vinyl)-[1,3]-dioxolan-[4R,5R]-4,5-dicarbonsäure,
2-(1-Methyl-vinyl)-[1,3]-dioxolan-[4S,5S]-4, 5-dicarbonsäure, 2-(1-(4,8,12-Trimethyl-tridecyl)-vinyl)-[1,3]-dioxolan-[4S,5S]-4, 5-dicarbonsäure, 2-(1-(4-Methyl-penta-3-enyl)-vinyl)-[1,3]-dioxolan-[4S,5S]-4,5-dicarbonsäure, 2-(1-(4,8-Dimethyl-nona-3,7-dienyl)-vinyl)-[1,3]-dioxolan-[4S,5S]-4, 5-dicarbonsäure oder 2-(1-(4,8,12-Trimethyl-trideka-3,7,11-trienyl)-vinyl)-[1,3]-dioxolan-[4S,5S]-4,5-dicarbonsäure.

Im ersten Schritt des erfindungsgemäßen Verfahrens wird die Dien-Komponente der allgemeinen Formel I mit der En-Komponente der allgemeinen Formel II im Sinne einer 4+2 Cycloaddition (Hetero-Diels-Alder Reaktion) umgesetzt.
Hetero-Diels-Alder Reaktionen von elektronenarmen En-Komponenten wie Acrylaten, Acroleinen, Allylalkoholen oder Crotonaten mit Acrolein oder an Position 1 oder 2 alkylierten Acroleinen sind bekannt.
So ist bekannt, daß sich äquimolare Mengen Methylvinylketon und Methyl-methacrylat unter (Hetero-)Diels-Alder Bedingungen (200°C, 2h) zu dem gemischten Diels-Alder Produkt (2-(Carboxymethyl)-2, 6-dimethyl-3,4-dihydro-2H-pyran) und dem Methylvinylketon-Dimer im Verhältnis 1:1 überführen lassen (B.P. Mundy, R.D. Otzenberger, A.R. DeBernadis, J. Org. Chem., 36, 1971, S. 2390). Neuere Untersuchungen bestätigen dieses Ergebnis. (J.G. Jun, D.W. Lee, Tetrahedron Lett. 1997, 38,S. 8207, letzte Zeile bis S. 8208, 2. Absatz). Ferner beschreiben B.P. Mundy et al. in obiger Schrift, daß die Umsetzung von Methylvinylketon mit in Position 2 methyliertem Acrolein (Methacrolein) nicht zum gewünschten Hetero-Diels-Alder-Produkt führt, während sich Methylvinylketon und Acrolein in das gewünschte Hetero-Diels-Alder-Produkt überführen lassen. Der Umsatz von 1-Methylacrolein mit 2-Methyl-allylalkohol führt über das Hetero-Diels-Alder-Produkt hinaus zum bicyclischen Naturstoff Frontalin. (B.P. Mundy, K.B. Lipkowitz, G.W. Dirks, Heterocycles 1977, 6, S. 64, Gleichung 11). Gore et al. konnten zeigen, daß sich 2-Methyl-1-penten-3-on und Methylcrotonat nicht zum gewünschten regioisomeren Hetero-Diels-Alder-Produkt umsetzten lassen, wohingegen der Umsatz von 2-Methyl-1-penten-3-on mit Methyl-methacrylat zum gewünschten regioisomeren Hetero-Diels-Alder-Produkt führt (W.E. Gore, G.T. Pearce, R.M. Silverstein, J. Org. Chem 1976, 41, S. 603-604). Ferner beschreiben Smith et al. weitere Hetero-Diels-Alder-Reaktionen von alkylierten Acroleinen mit alkylsubstituierten Alkyl-vinyl-ethern und Acrylaten (US 2,514,168; C.W. Smith, D.G. Norton, S.A. Ballard, J. Am. Chem. Soc. 1951, S. 5267-5272).
Es ist bekannt, daß die Einführung von Resten mit Elektronen-Donor-Eigenschaften in Position 2 von 1-Methyl-acrolein die Reaktionsfähigkeit von 1-Methyl-acrolein als Dien-Komponente einer Diels-Alder Reaktion stark herabsetzt (D.L. Boger, S.M. Weinreb, Hetero Diels-Alder - Methodology in Organic Synthesis, Academic Press, Inc, 1987, S. 185 f.). So ist nur ein Beispiel, die Diels-Alder-Dimerisierung von 3-Trimethylsilyl-oxybut-3-en-2-on (Position 3 von But-3-en-2-on entspricht Position 2 von 1-Methyl-Acrolein), bekannt, bei dem die Dimerisierung von Methylacrolein mit einem Silyloxyrest in Position 2 im Sinne einer Diels-Alder-Reaktion gelang. (S. Murai; I. Ryu, Y. Kadono, H. Katayama, K. Kondo, N. Sonoda, Chem. Lett. 1977, 1219 f.). Gemischte Diels-Alder-Reaktionen von Acroleinen, die in Position 2 einen Elektronen-Donor als Rest tragen mit Dienophilen sind nur in wenigen Fällen bekannt (D.L. Boger, S.M. Weinreb, loc. cit.). Dabei tragen die Acroleine in Position 2 einen Phenylthio-Rest und reagieren ausschließlich mit elektronenreichen En-Komponenten wie z. B. Ethylvinyl-ether (K. Takaki, M. Yamada, K. Negoro, J. Org. Chem. 1982, 47, 5246-5250). Funk et al. beschreiben eine gemischte, mit Yb katalysierte, Hetero-Diels-Alder Reaktion eines Acrolein mit einem Phenylacyloxyrest in Position 2 mit der elektronenreichen En-Komponente Ethylvinylether (J. Org. Chem. 1996, 61, 2599, Reaktion (e)).
Die erfindungsgemäße Umsetzung der oxyfunktionalisierten Dien-Komponente der Formel I mit der elektronenarmen En-Komponente II liefert die entsprechenden, bislang unbekannten 3,4-Dihydro-2H-Pyrane der allgemeinen Formel III.

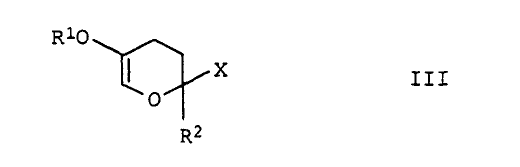

Die Durchführung der Reaktion kann unter den Reaktionsbedingungen der an sich bekannten Hetero Diels-Alder Reaktionen erfolgen (D.L. Boger, S.M. Weinreb, loc. cit.).
Die Umsetzung der beiden Komponenten kann rein thermisch, vorzugsweise unter Eigendruck, typischerweise bei Temperaturen von 100°C bis 250°C oder unter zusätzlicher Zugabe von Lewissäuren, wie Zinntetrachlorid, Bortrifluorid, Titantetrachlorid, Alkylaluminiumdihalogeniden, Dialkylaluminiumhalogeniden, Magnesiumhalogeniden sowie Halogeniden und Trifluormethansulfonaten der Lanthaniden erfolgen. Die Reaktion kann in organischen Lösungsmitteln, wie aliphatischen, aromatischen gegebenenfalls halogenierten Kohlenwasserstoffen oder Ethern oder auch in Substanz ohne Lösungsmittel durchgeführt werden. Bevorzugte organische Lösungsmittel sind Xylol, Toluol, Heptan, THF, Dioxan, Methylenchlorid oder Chloroform.
Die Dauer der Umsetzung beträgt typischerweise 6 bis 24 h. Die erhaltenen 3,4-Dihydro-2*H*-pyrane können in an sich bekannter Weise, beispielsweise durch thermische Trennverfahren wie Destillation aus der Reaktionslösung isoliert werden. Bei bevorzugten substituierten 3,4-Dihydro-2*H*-Pyrane der Formel III haben die Reste R¹, R² und X folgende Bedeutung:
- R¹: CH₃ oder COCH₃,
- R²: CH₃, 4,8,12-Trimethyl-tridecyl oder 4,8,12-Trimethyltrideka-3,7,11-trienyl und
- X: CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ oder CH(OCH₃)₂ oder eines der folgenden Strukturelemente X₁-X₇.

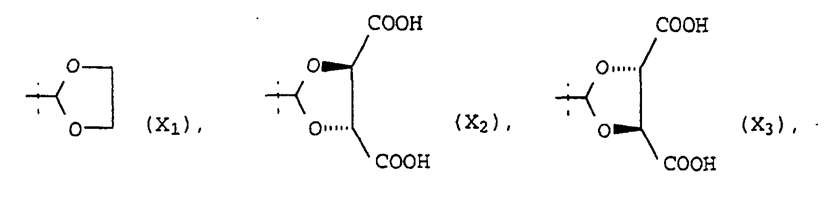

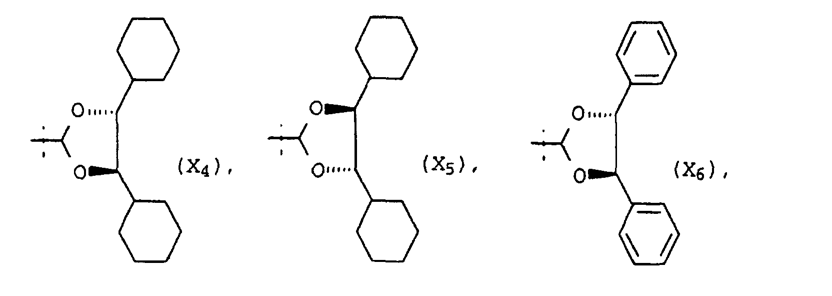

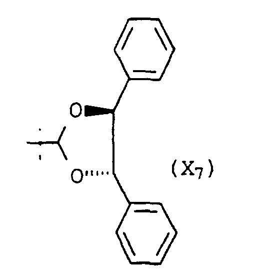

Bei den bevorzugten 3,4-Dihydro-2*H*-pyranen der Formel III sind beispielsweise folgende Reste kombiniert:

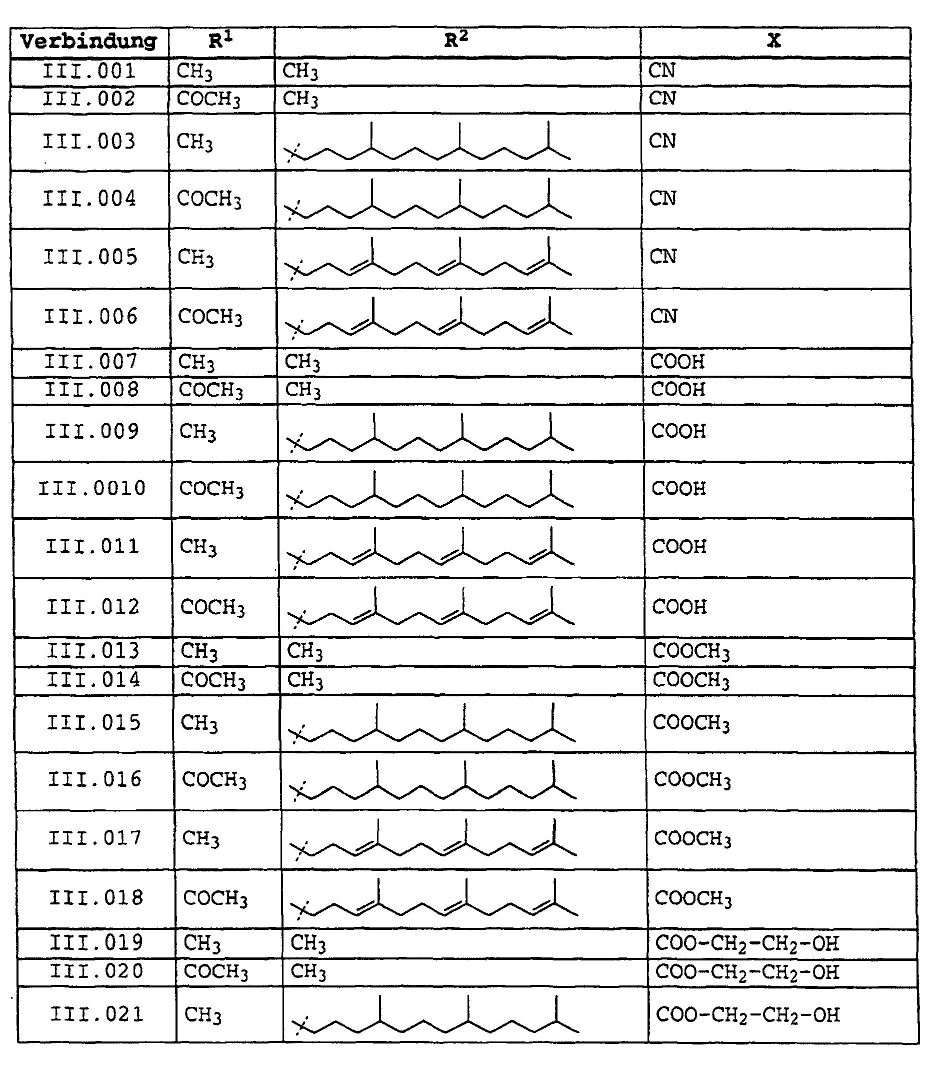

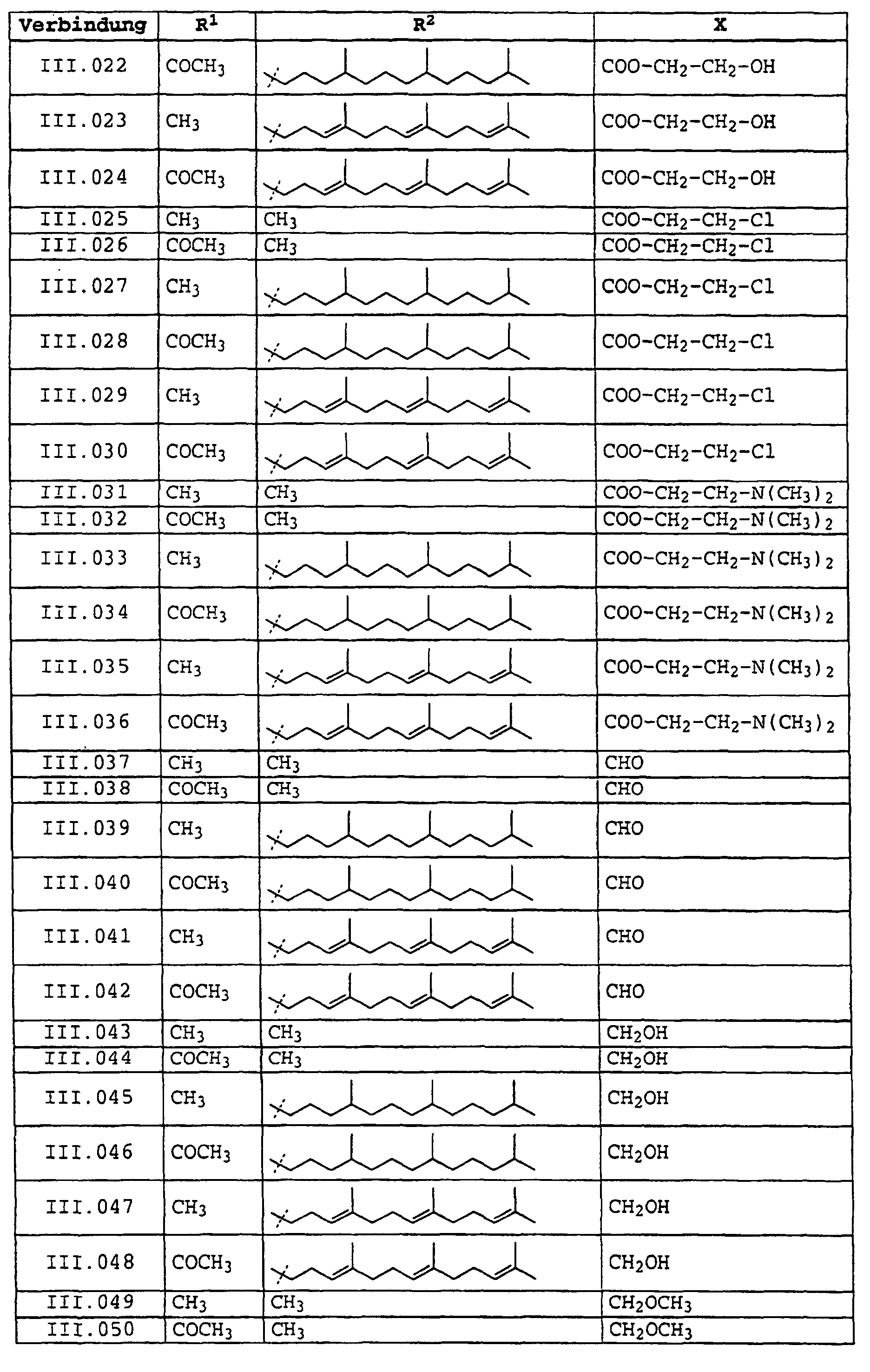

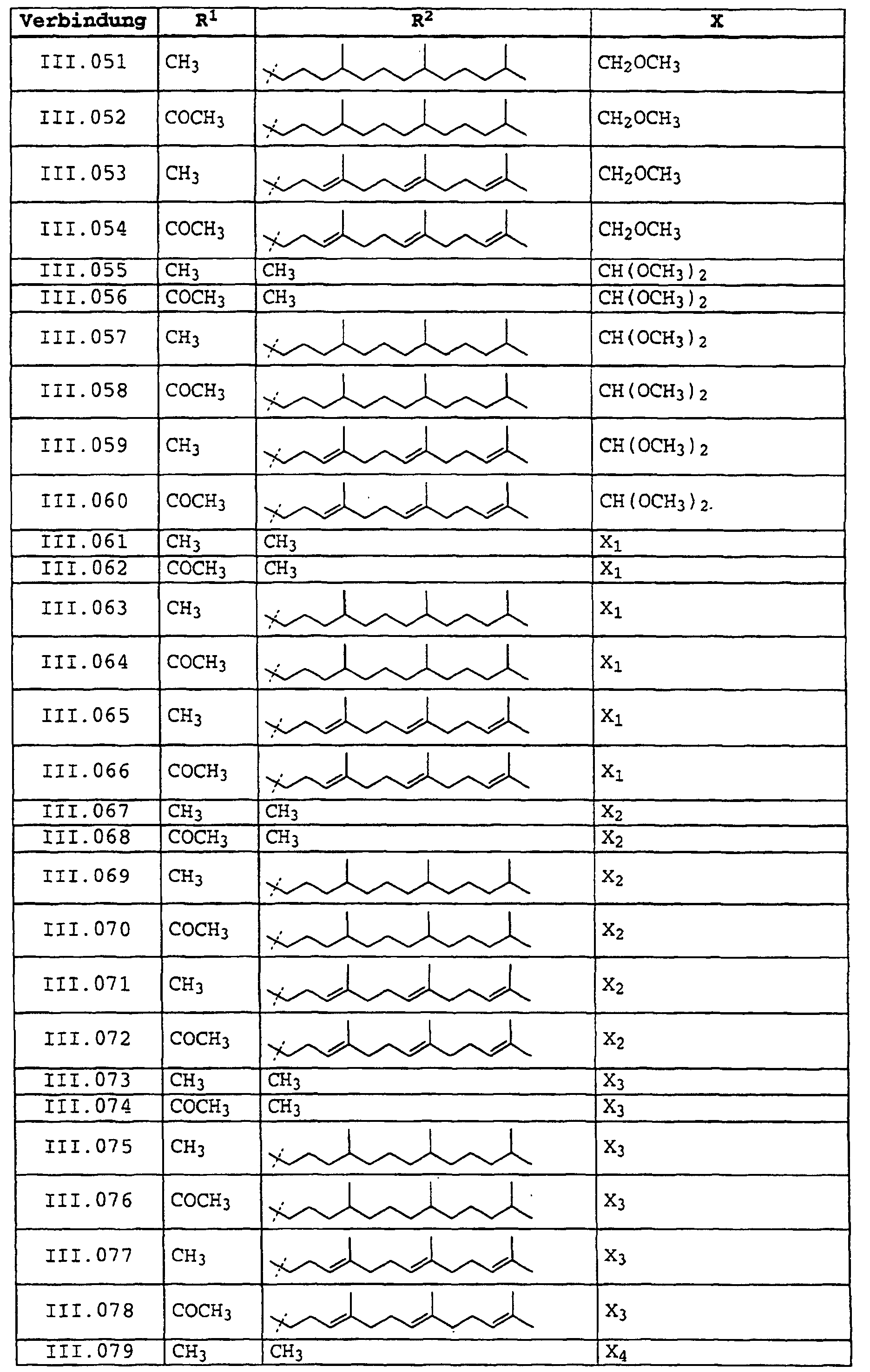

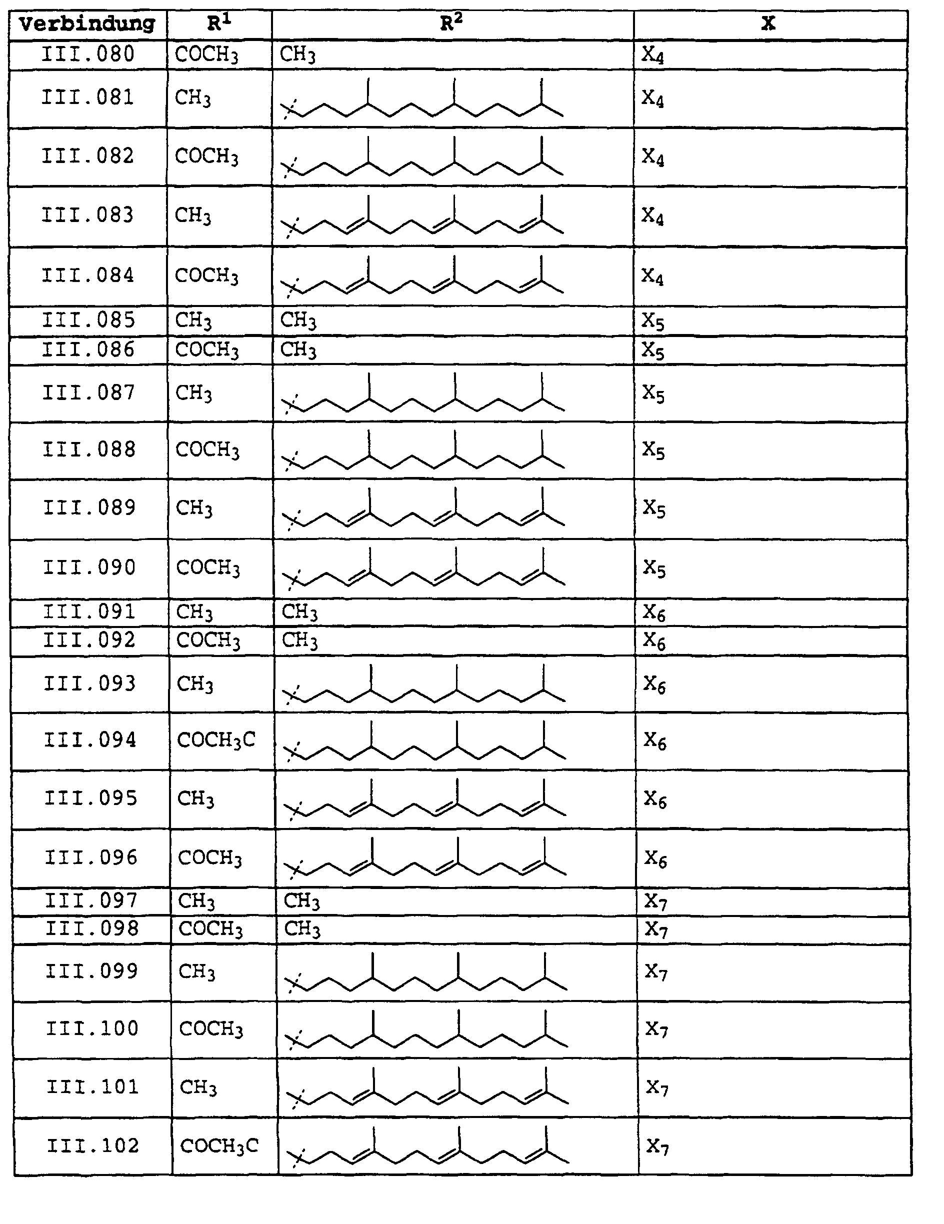

Im nächsten Schritt des erfindungsgemäßen Gesamtverfahrens werden die 3,4-Dihydro-2*H*-pyrane der allgemeinen Formel III mit einer Säure umgesetzt. Auf diese Weise wird unter Säurekatalyse der Rest R¹ abgespalten und das Enol freigesetzt, welches wiederum zum 5-Oxo-tetrahydropyran der allgemeinen Formel IV 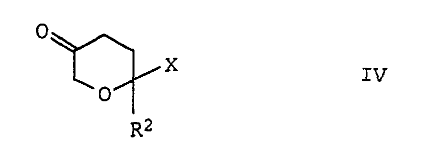 tautomerisiert.
Die Umsetzung der substituierten 3,4-Dihydro-2*H*-pyrane der allgemeinen Formel III mit einer Säure erfolgt in an sich bekannter Weise (J. March, Advanced Organic Chemistry, 4^{th} edition, 1992, J. Wiley & sons, S. 373 f.). Unter Säuren werden erfindungsgemäß Brönstedsäuren sowie deren wäßrige Lösungen verstanden. Beispielhaft seien Mineralsäuren wie beispielsweise HCl, H₂SO₄, HClO₄ und gegebenenfalls halogenierte, d.h. mit Cl, Br, I, F, substituierte Carbonsäuren wie Ameisensäure, Essigsäure, Propionsäure, Chloressigsäure, Trifluoressigsäure genannt.
Typischerweise sind zum Überführen von Enolether in Enole wäßrige Säurelösungen mit niedriger Säurekonzentration, wie 0,01-12 M ausreichend. Beispielhaft sei eine 2M-HCl Lösung erwähnt.
Die Reaktion wird in einem organischen Lösungsmittel, wie vorstehend erwähnt, bevorzugt in Methylenchlorid durchgeführt. Die Dauer und Temperatur der Reaktion richtet sich nach dem Rest R¹. Typischerweise wird die Reaktion bei Raumtemperatur 2 bis 12 h durchgeführt.

Bei bevorzugten, substituierten 5-Oxo-tetrahydropyranen der allgemeinen Formel IV haben die Reste R² und X folgende Bedeutung:
- R²: CH₃, 4,8,12-Trimethyl-tridecyl oder 4,8,12-Trimethyltrideka-3,7,11-trienyl und
- X: CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ oder CH(OCH₃)₂ oder eines der Strukturelemente X₁-X₇, wie vorstehend für die substituierten 3,4-Dihydro-2H-Pyrane der Formel III aufgeführt.

Bei den bevorzugten substituierten 5-Oxo-tetrahydropyranen der allgemeinen Formel IV sind beispielsweise folgende Reste kombiniert:

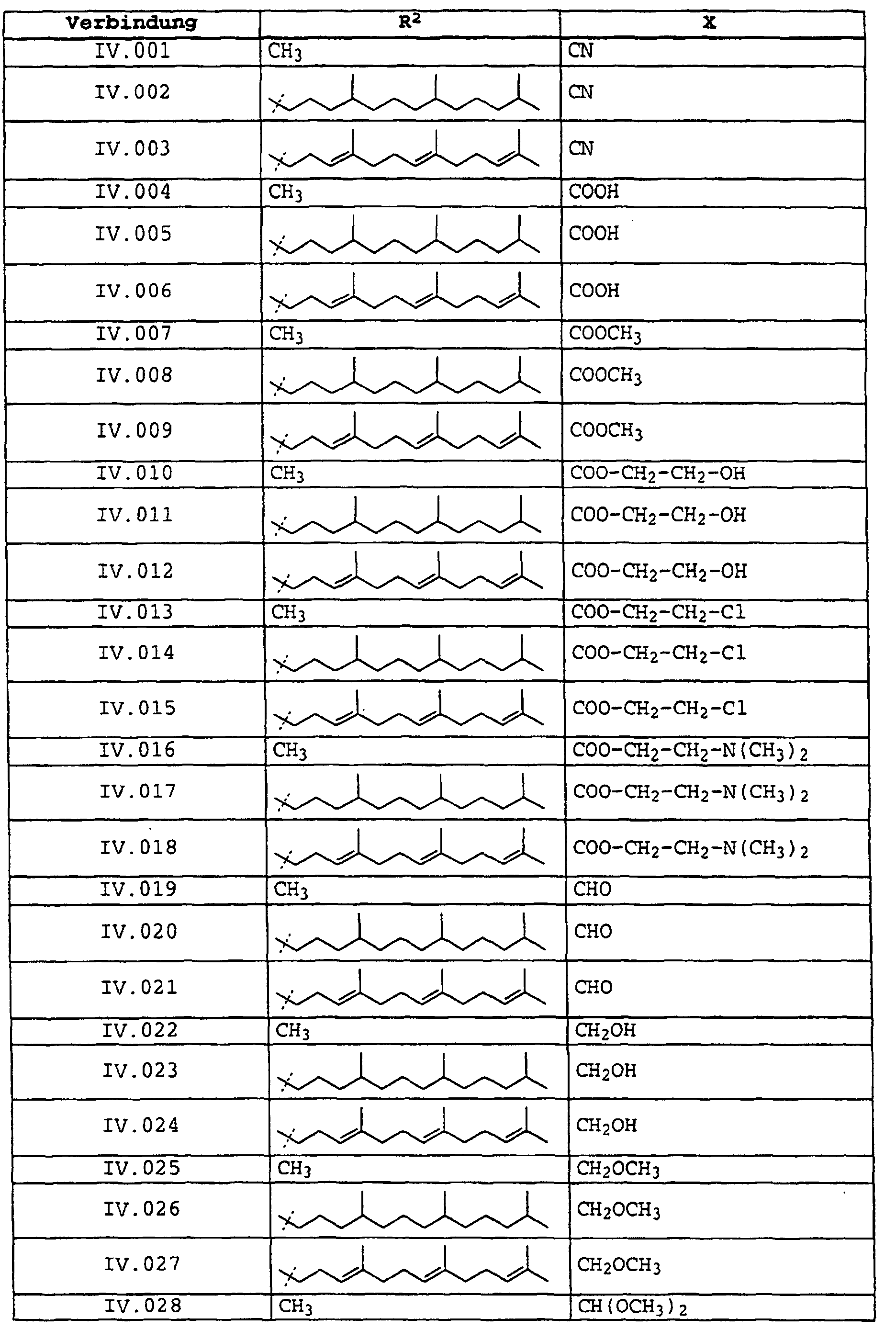

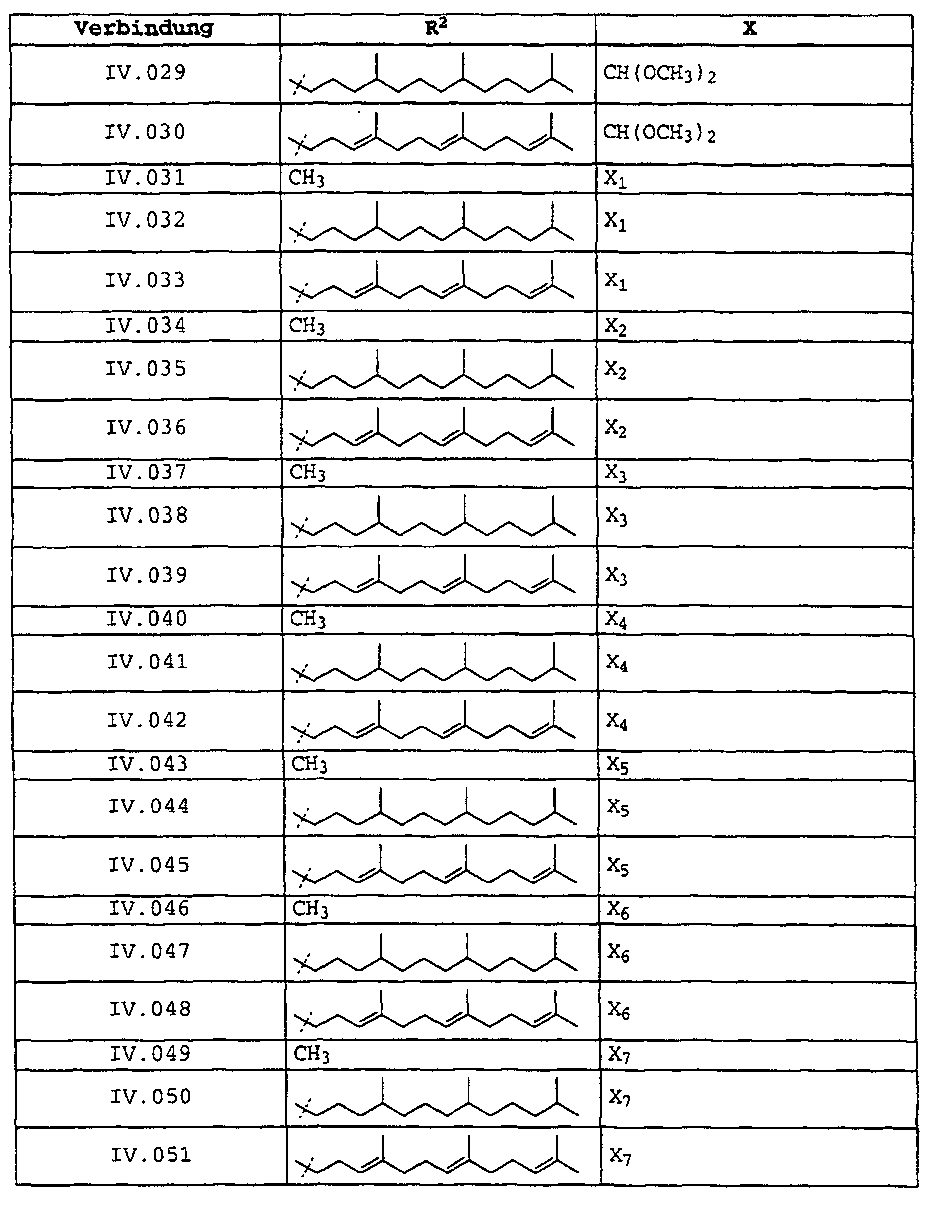

Im nächsten Schritt des Gesamtverfahrens werden die 5-Oxo-tetrahydropyrane der allgemeinen Formel IV mit substituierten Vinylketonen der allgemeinen Formel V im Sinne einer Robinson-Anellierung umgesetzt(J. March, Advanced Organic Chemistry, 4^{th} edition, 1992, J. Wiley & sons, S. 943-944)

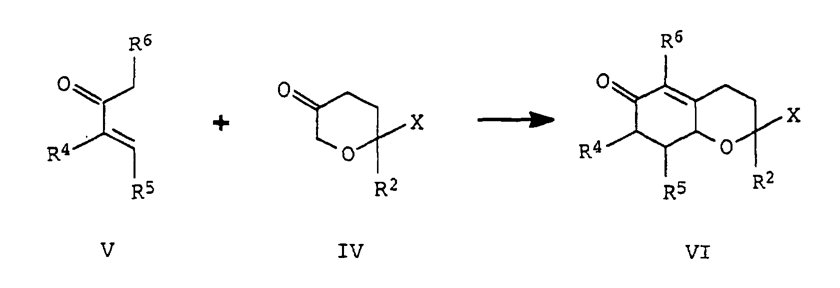

Die Reste R⁴, R⁵ und R⁶ bedeuten unabhängig voneinander Wasserstoff oder einen C₁-C₄-Alkylrest, wie vorstehend für R¹ beschrieben, insbesondere Methyl. Bevorzugte substituierte Vinylketone der allgemeinen Formel V sind
4-Methyl-hex-4-en-3-on (R⁴ = R⁵ = R⁶ = Methyl),
Hex-4-en-3-on (R⁴ = Wasserstoff; R⁵ = R⁶ = Methyl),
3-Methyl-pent-3-en-2-on (R⁴ = R⁵ = Methyl, R⁶ = Wasserstoff) und Pent-3-en-2-on (R⁴ = R⁶ = Wasserstoff, R⁵ = Methyl).

Die Durchführung der Robinson Anellierung kann in an sich bekannter Weise erfolgen (M.E. Jung, Tetrahedron 1976, 32, 3-31; T. Sato et al. Tetrahedron Letters 1990, 31, 1581-1584 und darin zitierte Schriften sowie F. Eicher, K. Wanner, Arch. der Pharmazie 1984, 317, 958-962).
In einer bevorzugten Ausführungsform erfolgt die Umsetzung nach der an sich bekannten Enamin-Methode (G. Storck et al., J. Am. Chem. Soc. 1963, 85, 207). Dabei werden zunächst die substituierten 5-Oxo-tetrahydropyrane der allgemeinen Formel IV mit sekundären Aminen der Formel VIII

HN(R⁹)₂ VIII

zu den entsprechenden Enaminen der Formel IX 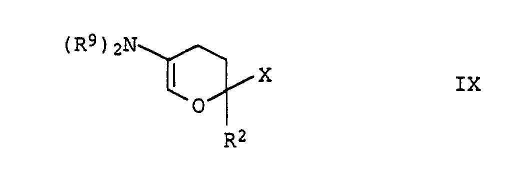 umgesetzt. Beide Reste R⁹ stellen einen gleichen oder verschiedenen C₁-C₄-Alkylrest, wie vorstehend für R¹ erwähnt, vorzugsweise Methyl oder Ethyl, oder einen Phenylrest oder beide Reste R⁹ einen, im Sinne eines cyclischen Amins verbrückenden C₂-C₆-Alkylenrest, wie Ethylen, Propylen, Butylen, Pentylen, Neopentylen oder Hexylen oder einen C₂-C₆-Alkylenrest, wobei eine oder zwei Methylengruppen durch Sauerstoff ersetzt sind, wie -CH₂-O-CH₂- oder -CH₂-CH₂-O-CH₂-CH₂-, dar.
Bevorzugte sekundäre Amine der Formel VIII sind dementsprechend Amine, wie Dimethylamin, Diethylamin, Pyrrolidin oder Morpholin, wobei Pyrrolidin oder Morpholin besonders bevorzugt sind.

Dabei setzt man die substituierten 5-Oxo-tetrahydropyrane der allgemeinen Formel IV in einem organischen Lösungsmittel, wie vorstehend im Fall der Umsetzung zur Verbindung der Formel III erwähnt, zunächst mit dem sekundären Amin der Formel VIII und einem wasserbindenden Trockenmittel, wie beispielsweise MgSO₄, Na₂SO₄ oder TiCl₄ zum Enamin der Formel IX um. In einer weiteren Ausführungsform kann das gebildete Wasser über azeotrope Destillation durch Zusatz von Schleppern, wie beispielsweise Benzol, Toluol, Pentan oder CH₂Cl₂ entfernt werden. Die Temperatur der Reaktion ist unkritisch und beträgt bei Verwendung von Trocknungsmitteln typischerweise Raumtemperatur. Die Dauer der Reaktion beträgt typischerweise 12 bis 24 h.
Anschließend werden die aus IV gebildeten Enamine der Formel IX mit den substituierten Vinylketonen der Formel V 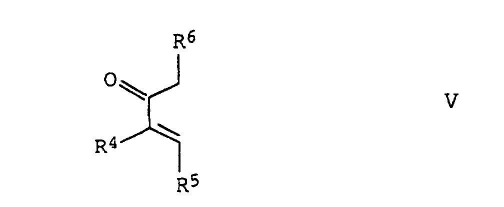 zu den entsprechenden Keto-Enaminen der Formel X 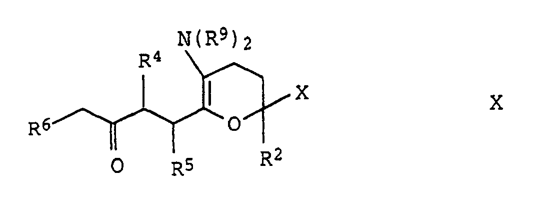 in an sich bekannter Weise im Sinne einer Michael-Addition umgesetzt (Nelson et al., J. Org. Chem. 1969, 34, 1225).

Anschließend werden die Keto-Enamine der Formel X durch Zugabe von Säuren, beispielsweise Mineralsäuren wie Salzsäure oder Schwefelsäure, bzw. deren wäßrige Lösungen zu den entsprechenden Diketon-Verbindungen der Formel XI 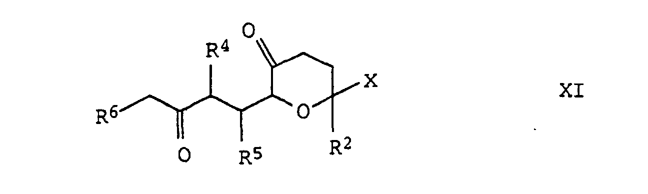 umgesetzt, und die intermediär auftretenden Diketone der Formel XI durch Zugabe einer Base, wie beispielsweise Diisopropylethylamin, Lithiumdiethylamid oder C₁-C₄-Natriumalkoholate, wie Natriummethylat oder Natriumethylat in an sich bekannter Weise, in die substituierten Chromen-6-on-Derivate der allgemeinen Formel VI überführt (M. E. Jung, Tetrahedron 1976, 32, 3; R.E. Ganley, Synthesis 1976, 777)
Bei bevorzugten, substituierten Chromen-6-on-Derivate der allgemeinen Formel VI haben die Reste R², R⁴, R⁵, R⁶ und X folgende Bedeutung:
- R²: CH₃, 4,8,12-Trimethyl-tridecyl oder 4,8,12-Trimethyltrideka-3,7,11-trienyl,
- R⁴, R⁵ und R⁶: unabhängig voneinander Wasserstoff oder CH₃ und
- X: CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ oder CH(OCH₃)₂ oder eines der Strukturelemente X₁-X₇, wie vorstehend für die substituierten 3,4-Dihydro-2*H*-Pyrane der Formel III aufgeführt.

Bei den bevorzugten Chromen-6-on-Derivaten der Formel VI sind beispielsweise folgende Reste kombiniert:

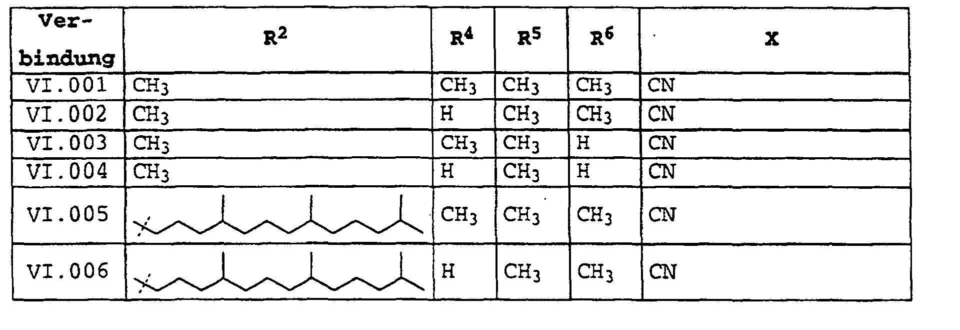

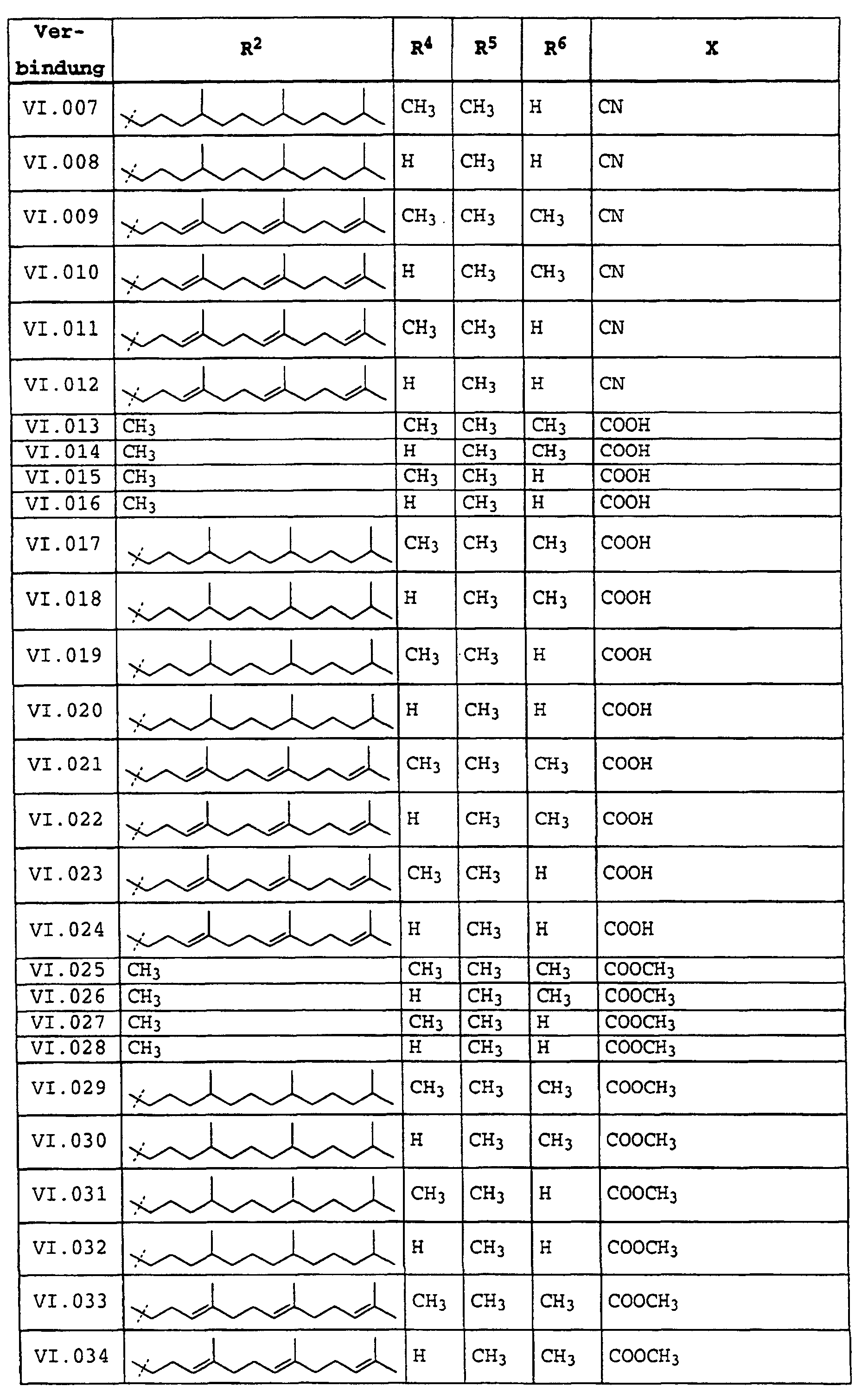

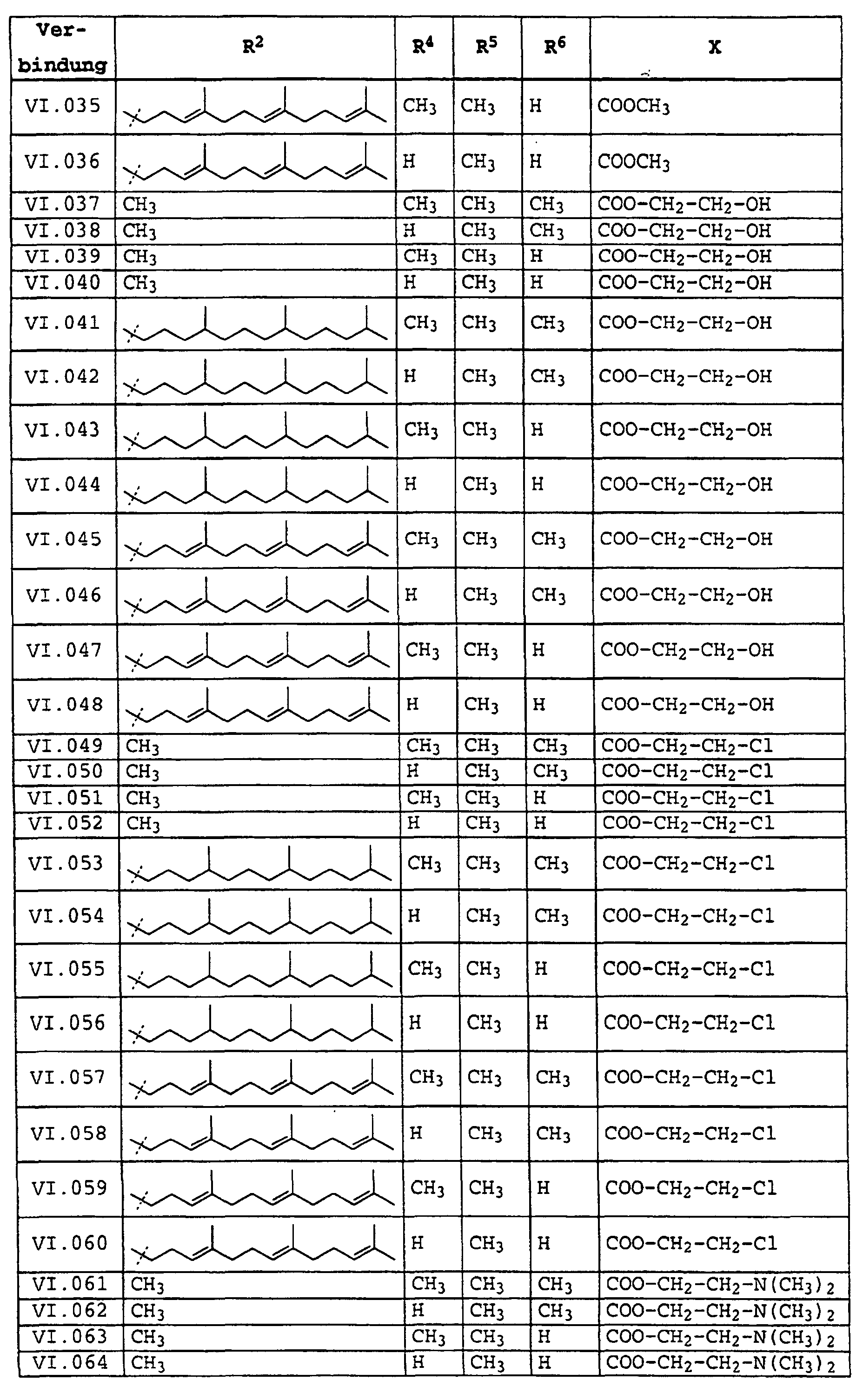

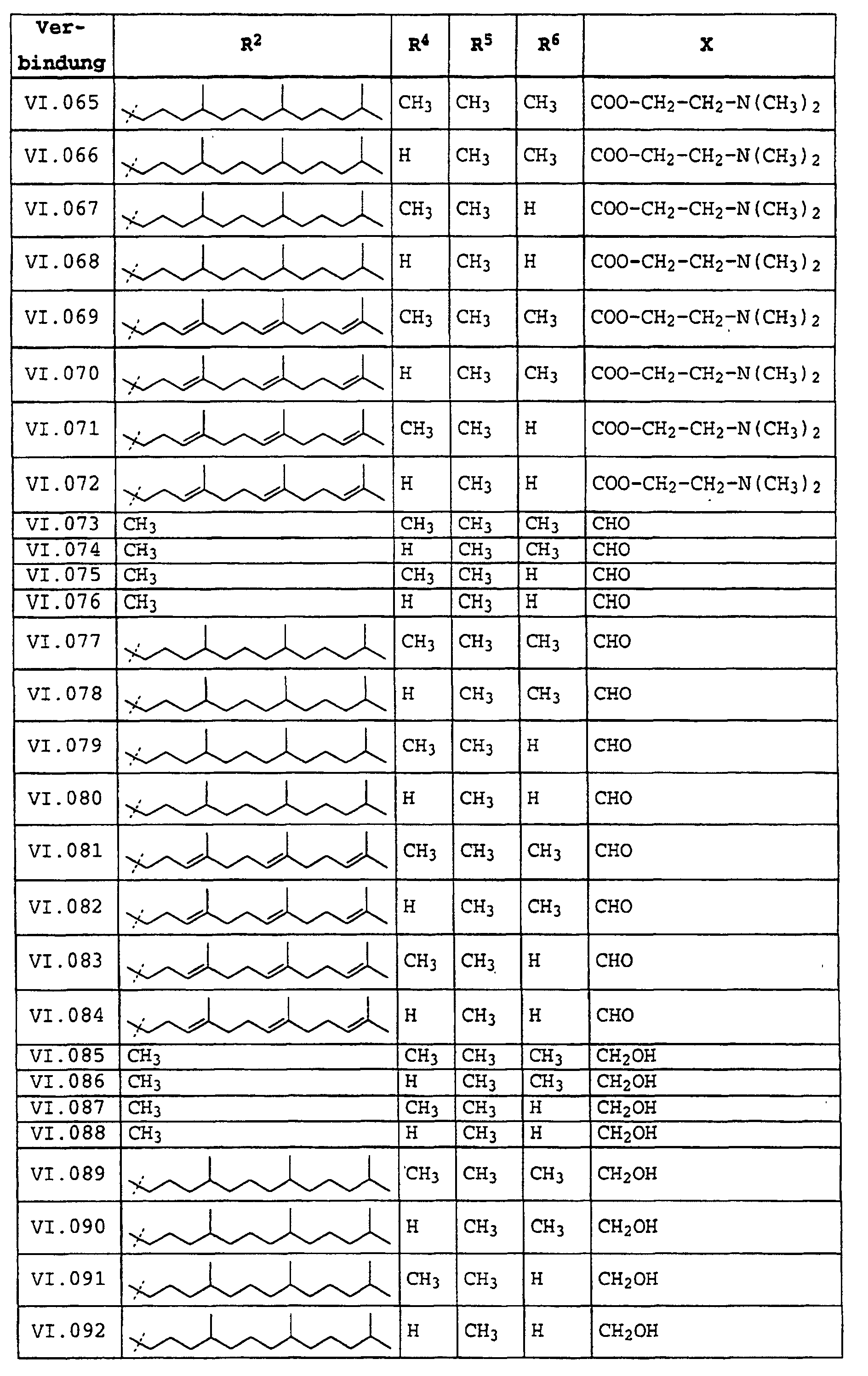

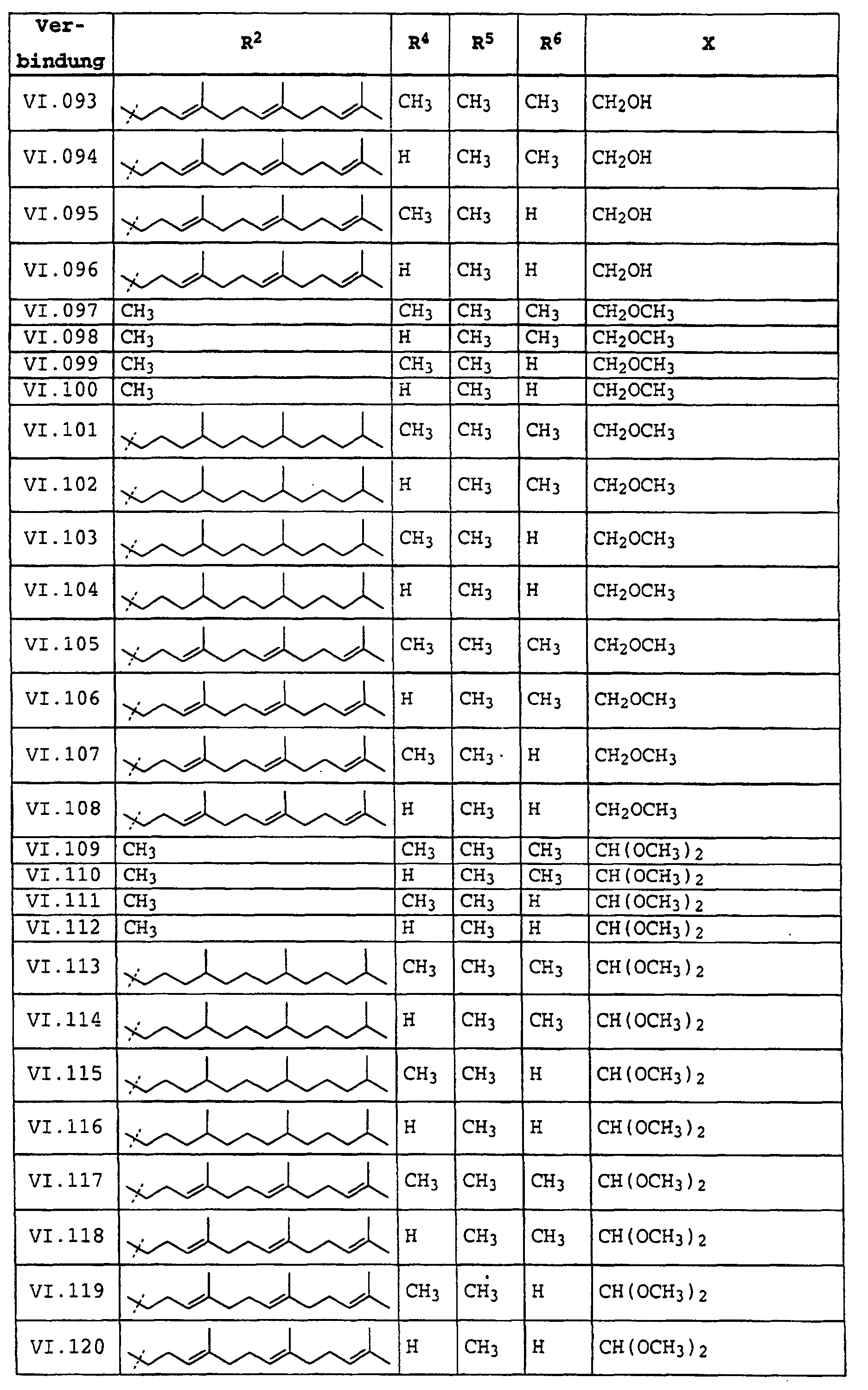

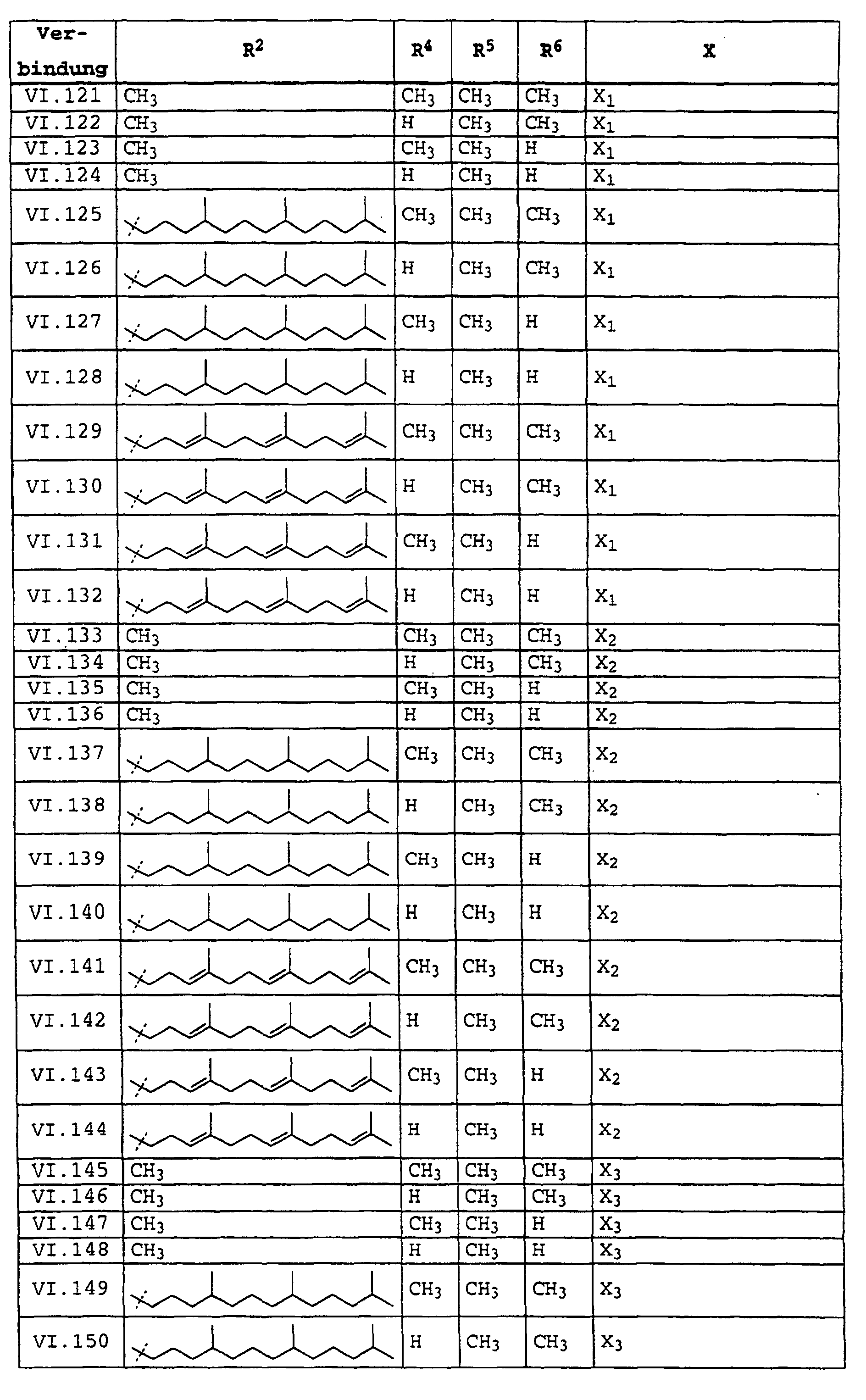

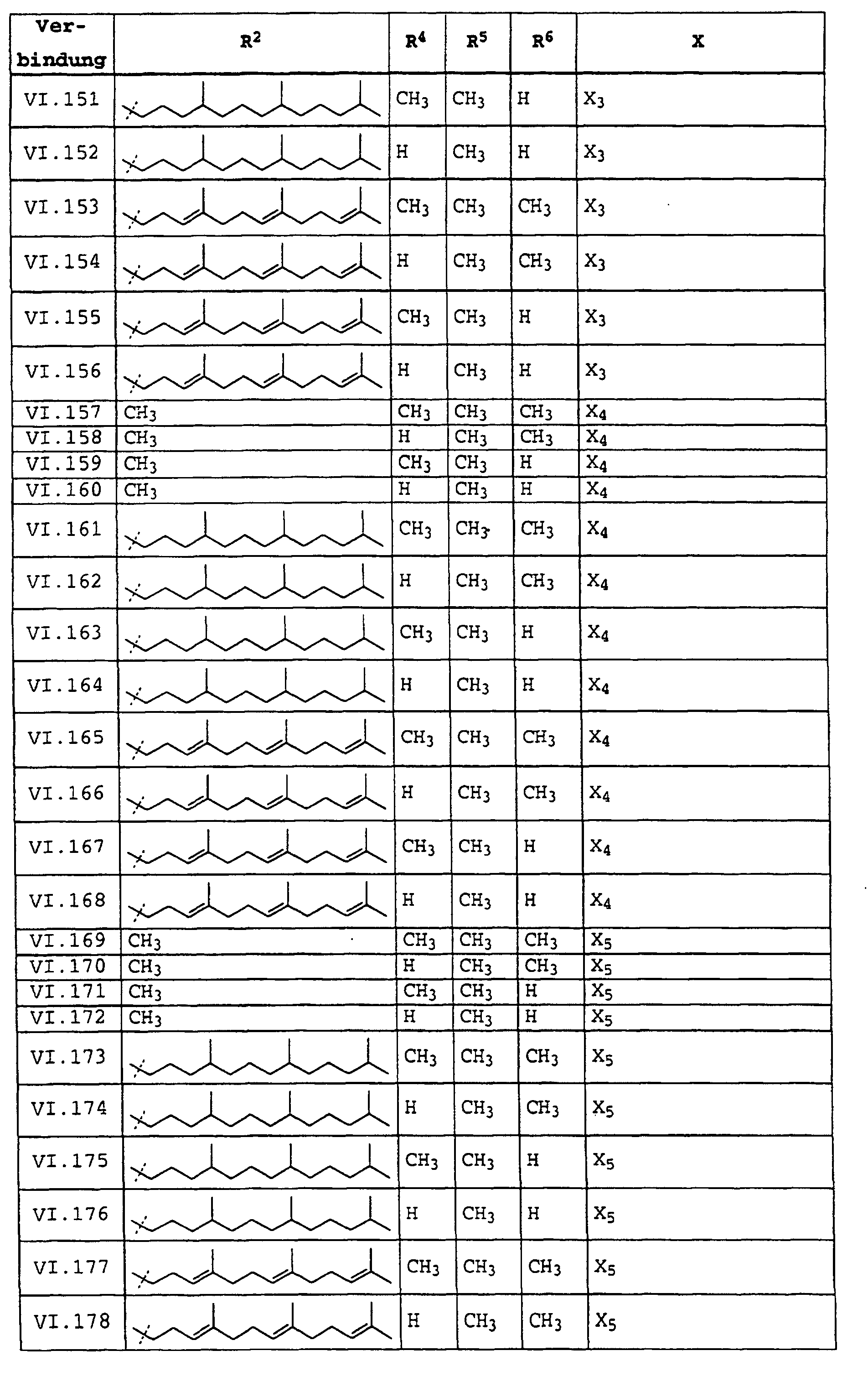

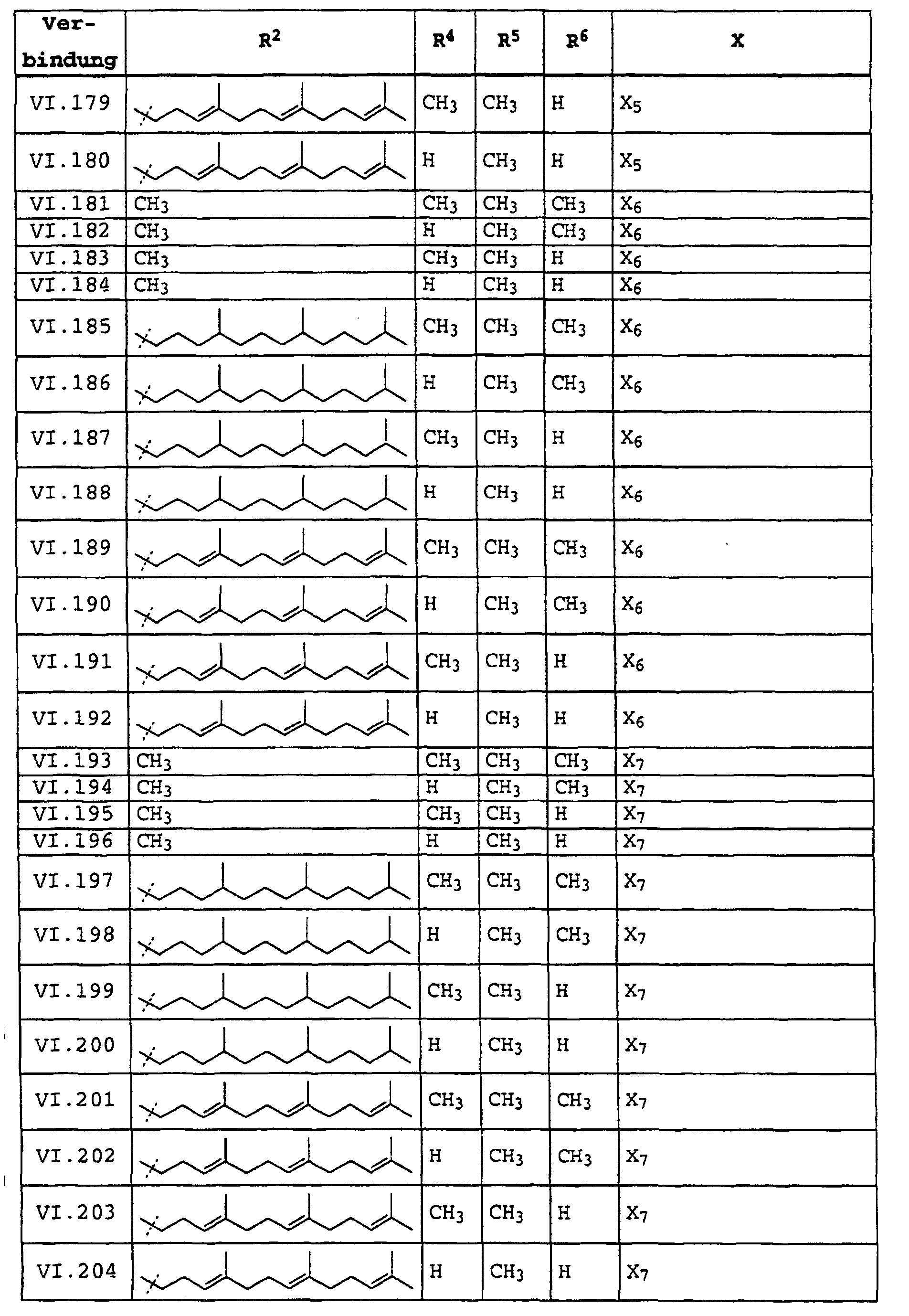

Im letzten Schritt des erfindungsgemäßen Verfahrens werden die Chromen-6-on-Derivaten der allgemeinen Formel VI durch Zugabe von dehydrierenden Agenzien wie Pd(C), MnO₂, S, Se oder DDQ (Dichlordicyanoquinon; 4,5-Dichlor-3,6-dioxo-cyclohexa-1,4-dien-1,2-dicarbonitril) in an sich bekannter Weise zu den substituierten Chromanen der allgemeinen Formel VII dehydriert (R.P. Fu, R.G. Harvey, Chem. Rev. 1978, 78, 317).

Das erfindungsgemäße Verfahren stellt ein neues Verfahren zur Herstellung von Chromanderivaten der allgemeinen Formel VII dar. Dabei nutzt das Verfahren wohlfeile, preiswerte Ausgangsmaterialien. Des weiteren umgeht das Verfahren das bekannte Problem der Regioselektivität bei elektrophilen Substitutionen an elektronenreichen Aromaten bei Verfahren nach dem Stand der Technik und ermöglicht den Aufbau eines flexiblen Substituentenmusters am aromatischen Teil des Chromangerüstes. Zudem führt das Verfahren über neue, leicht handhabbare Zwischenverbindungen, die ihrerseits wertvolle Zwischenverbindungen für neue pharmazeutische Wirkstoffe, Aroma- und Riechstoffe sowie Vitamine und Nahrungszusatzstoffe darstellen.

Die nachstehenden Beispiele erläutern die Erfindung.

### Beispiel 1

### Herstellung von 2-Methyl-2-methoxycarbonyl-5-methoxy-3,4-dihydro-2H-pyran (IIIa)

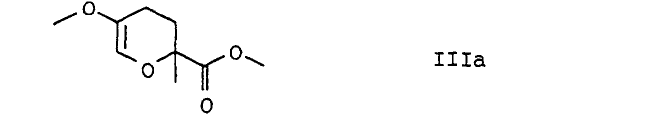

### 1a) Herstellung ohne Zusatz von Lewissäuren

0,2 mol α-Methoxyacrolein wurden in 100 ml Xylol gelöst und anschließend 0,2 mol Methacryl-säuremethylester zugegeben. Das Gemisch wurde 12 h bei 180°C unter Eigendruck gerührt. Nach Vakuumdestillation des Ansatzes wurden 0,08 mol (40 % d.Th.) des Produktes 2-Methyl-2-methoxycarbonyl-5-methoxy-3,4-dihydro-2*H*-pyran als farbloses Öl isoliert.
MS(EI): 186 (M+, 48 %) ,155 (33%), 127 (62 %)
¹³C-NMR: 174,5, 140,5, 122,9, 76,1, 55,0, 52,4, 29,8, 25,1, 19,9

### 1b) Herstellung unter Zusatz von Lewis-Säuren

0,2 mol α-Methoxyacrolein wurden in 100 ml Xylol gelöst. Anschließend wurden unter Kühlung 10 mmol Zinntetrachlorid und danach 0,2 mol Methacryl-säuremethylester zugegeben und das Gemisch 12 h bei 120°C unter Eigendruck gerührt. Nach Vakuumdestillation des Ansatzes wurden 32 % d.Th. des Produktes 2-Methyl-2-methoxycarbonyl-5-methoxy-3,4-dihydro-2H-pyran als schwachgelbes Öl isoliert. Physikalische Daten siehe 1a.

### Beispiel 2

### Herstellung von 2-Methyl-2-formyl-5-methoxy-3,4-dihydro-2Hpyran (IIIb)

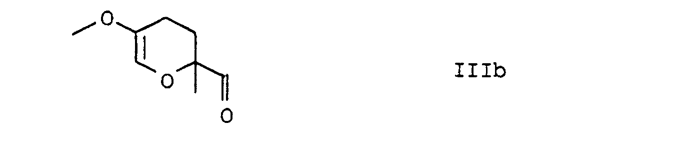

0,2 mol α-Methoxyacrolein wurden in 100 ml Xylol gelöst und anschließend 0,2 mol Methacrolein zugegeben. Das Gemisch wurde 12 h bei 180°C unter Eigendruck gerührt. Nach Vakuumdestillation des Ansatzes werden 0,11 mol (55 % d.Th.) des Produktes 2-Methyl-2-formyl-5-methoxy-3,4-dihydro-2*H*-pyran als farbloses Öl isoliert.
MS(CI): 156 (M⁺, 12 %), 141 (14 %), 87 (100 %)

### Beispiel 3

### Herstellung von 2-Methyl-2-hydroxymethyl-5-methoxy-3,4-dihydro-2H-pyran (IIIc)

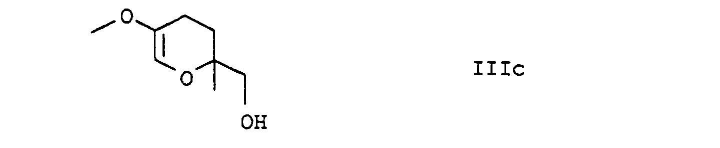

0,2 mol α-Methoxyacrolein wurden in 100 ml Xylol gelöst und anschließend 0,2 mol Methallyl-alkohol zugegeben. Das Gemisch wurde 12 h bei 180°C unter Eigendruck gerührt. Nach Vakuumdestillation des Ansatzes wurden 0,15 mol (75 % d.Th.) des Produktes 2-Methyl-2-hydroxymethyl-5-methoxy-3,4-dihydro-2*H*-pyran als farbloses Öl isoliert.
MS(CI): 158 (M+, 4 %), 141 (9 %), 127 (33 %), 87 (100 %)

### Beispiel 4

### Herstellung von 2-Methyl-5-methoxy-3,4-dihydro-2H-pyran-2-carbonsäure-2-Hydroxyethylester (IIId)

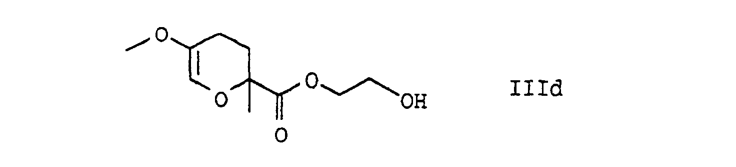

0,2 mol α-Methoxyacrolein wurden in 100 ml Xylol gelöst und anschließend 0,2 mol Methacrylsäure-2-hydroxyethylester zugegeben. Das Gemisch wurde 12 h bei 180°C unter Eigendruck gerührt. Nach Vakuumdestillation des Ansatzes wurde das Produkt 2-Methyl-5-methoxy-3,4-dihydro-2*H*-pyran-2-carbonsäure-2-Hydroxyethylester als farbloses Öl mit einer Ausbeute von 48% isoliert.
MS(EI): 216 (M+, 88 %), 127 (100 %), 185 (10 %), 154 (9 %)

### Beispiel 5

### Herstellung von 2-Methyl-5-methoxy-3,4-dihydro-2H-pyran-2-carbonsäure-2-chlorethylescer (IIIe)

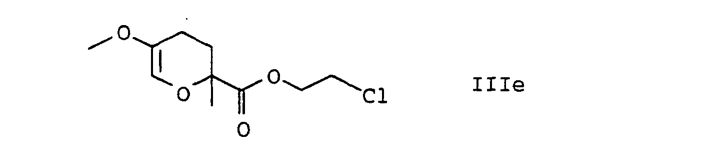

0,2 mol α-Methoxyacrolein wurden in 100 ml Xylol gelöst und anschließend 0,2 mol Methacrylsäure-2-chloroethylester zugegeben. Das Gemisch wurde 12 h bei 180°C unter Eigendruck gerührt. Nach Vakuumdestillation des Ansatzes wurde das Produkt 2-Methyl-5-methoxy-3,4-dihydro-2*H*-pyran-2-carbonsäure-2-chlorethylester als farbloses Öl mit einer Ausbeute von 45 % isoliert.
MS(EI): 234 (M+, 12 %, 199 (27 %), 113, (100 %)

### Beispiel 6

### Herstellung von 2-Methyl-5-methoxy-3,4-dihydro-2H-pyran-2-carbonsäure-2-dimethylaminoethylester (IIIf)

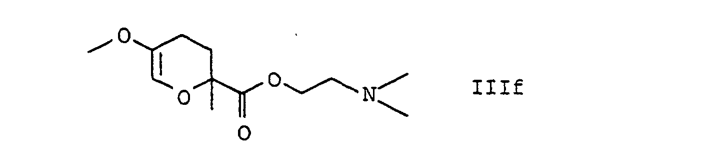

0,2 mol α-Methoxyacrolein wurden in 100 ml Xylol gelöst und anschließend 0,2 mol Methacrylsäure-2-dimethylaminoethylester zugegeben. Das Gemisch wurde 12 h bei 180°C unter Eigendruck gerührt. Nach Säulenchromatographie des Ansatzes wurde das Produkt 2-Methyl-5-methoxy-3,4-dihydro-2*H*-pyran-2-carbonsäure-2-dimethylaminoethylester mit einer Ausbeute von 52 % isoliert.
NMR:δ(¹³C) in [ppm]: 198,1; 173,6; 140,5; 123,1; 62,6; 57,2; 55,1; 45,2; 29,8; 24,5; 21,6

### Beispiel 7

### Herstellung von 2-Methyl-5-oxo-tetrahydropyran-2-carbonsäure-2-chlorethylester (IVe) durch Hydrolyse von 2-Methyl-5-methoxy-3,4-dihydro-2H-pyran-2-carbonsäure-2-chlorethylester (IIIg)

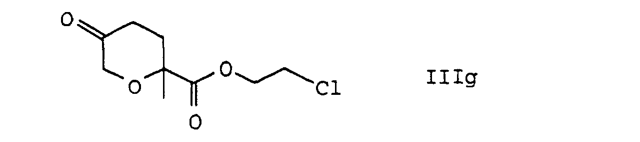

3,72 g (20 mmol) 2-Methyl-5-methoxy-3,4-dihydro-2*H*-pyran-2-carbonsäure-2-chloroethylester (IIIe) wurden in 20 ml Methylenchlorid gelöst, bei RT mit 20 ml wässriger HCl-Lösung (2M) versetzt und gerührt, bis nach DC-Kontrolle kein Edukt mehr zu erkennen war (4 h). Die Phasen wurden getrennt. Nach Trocknung der organischen Phase und Entfernen des Solvens verbleiben 3,20 g (74 %) 2-Methyl-5-oxo-tetrahydropyran-2-carbonsäure-2-chlorethylester (IVe) als gelbes Öl. MS(EI): 220 (M⁺, 10 %), 151 (4 %), 113 (100 %)

## Patentansprüche

1. Verfahren zur Herstellung substituierter Chromanderivate der allgemeinen Formel VII, 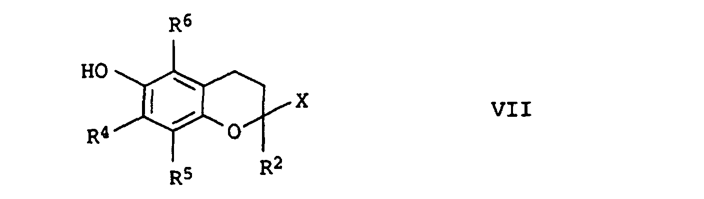 wobei
X die Gruppe -CN, -COOR³, -CHO, -CH₂OR⁷ oder -CH(OR⁸)₂,
R² einen C₁-C₂₃-Alkyl-, C₂-C₂₃-Alkenyl, C₆-C₁₈-Aryl-, oder C₇-C₁₈-Aralkyl-Rest,
R³ Wasserstoff, einen C₁-C₄-Alkylrest, einen C₁-C₄-Halogenalkylrest, einen C₁-C₄-Hydroxyalkylrest oder einen C₁-C₄-Aminoalkylrest,
R⁴, R⁵, R⁶ unabhängig voneinander Wasserstoff oder einen C₁-C₄-Alkylrest,
R⁷ Wasserstoff oder einen C₁-C₄-Alkylrest,
R⁸ einen C₁-C₄-Alkylrest, oder beide Reste einen C₂-C₆-Alkylenrest bedeuten, der die beiden Sauerstoffatome im Sinne eines cyclischen Acetals verbrückt und gegebenenfalls verzweigt ist oder ein bis zwei Carboxylgruppen, Cyclohexyl- oder Phenylreste tragen kann
darstellen, **dadurch gekennzeichnet, daß** man in einem ersten Schritt substituierte Acroleine der allgemeinen Formel I, 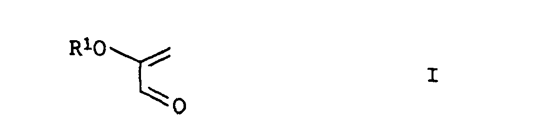 wobei
R¹ einen C₁-C₄-Alkyl-, C₆-C₁₈-Aryl-, C₇-C₁₈-Aralkyl-, C₁-C₄-Acyl-, einen halogenierten C₁-C₄-Acylrest oder eine andere säurelabile Schutzgruppe der Hydroxyfunktion darstellt,
mit Acrylnitrilen, Acrylaten, Acroleinen, Acroleinacetalen, Allylalkoholen oder Allylethern der allgemeinen Formel II 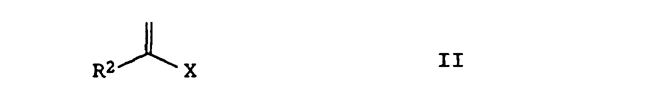 zu den entsprechenden 3,4-Dihydro-2*H*-Pyranen der allgemeinen Formel III 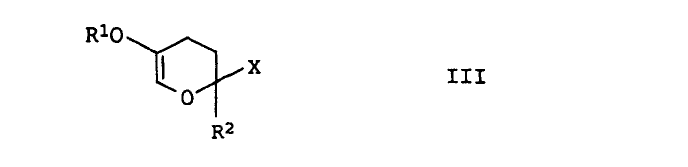 umsetzt und diese in einem zweiten Schritt mit einer Säure zu den entsprechenden 5-Oxo-tetrahydropyranen der allgemeinen Formel IV 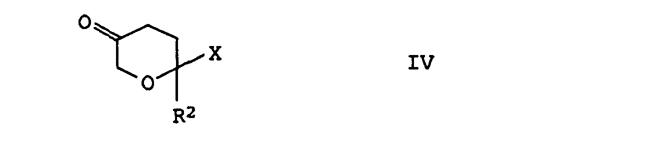 umsetzt und diese in einem dritten Schritt mit substituierten Vinylketonen der allgemeinen Formel V 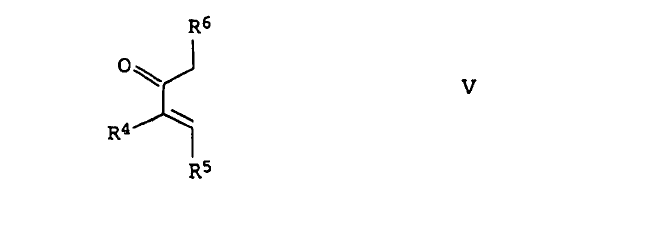 zu den entsprechenden Chromen-6-on-Derivaten der allgemeinen Formel VI 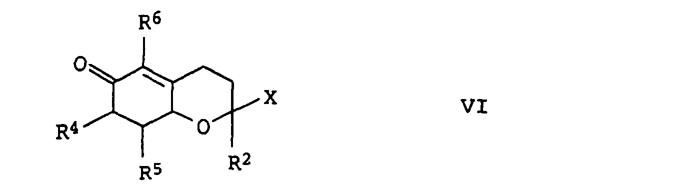 umsetzt und diese in einem 4 Schritt zu den substituierten Chromanderivaten der allgemeinen Formel VII dehydriert.

2. Substituierte 3,4-Dihydro-2*H*-Pyrane der allgemeinen Formel III, 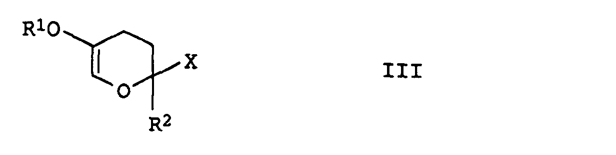 wobei
R¹ einen C₁-C₄-Alkyl-, C₆-C₁₈-Aryl-, C₇-C₁₈-Aralkyl-, C₁-C₄-Acyl-, einen halogenierten C₁-C₄-Acylrest oder eine andere säurelabile Schutzgruppe der Hydroxyfunktion darstellt,
X die Gruppe -CN, -COOR³, -CHO, -CH₂OR⁷ oder -CH(OR⁸)₂,
R² einen C₁-C₂₃-Alkyl-, C₂-C₂₃-Alkenyl, C₆-C₁₈-Aryl-, oder C₇-C₁₈-Aralkyl-Rest,
R³ Wasserstoff, einen C₁-C₄-Alkylrest, einen C₁-C₄-Halogenalkylrest, einen C₁-C₄-Hydroxyalkylrest oder einen C₁-C₄-Aminoalkylrest,
R⁷ Wasserstoff oder einen C₁-C₄-Alkylrest,
R⁸ einen C₁-C₄-Alkylrest, oder beide Reste einen C₂-C₆-Alkylenrest bedeuten, der die beiden Sauerstoffatome im Sinne eines cyclischen Acetals verbrückt und gegebenenfalls verzweigt ist oder ein bis zwei Carboxylgruppen, Cyclohexyl- oder Phenylreste tragen kann,
bedeuten.

3. Substituierte 3,4-Dihydro-2*H*-Pyrane der Formel III nach Anspruch 2, wobei die Substituenten folgende Bedeutung haben:
R¹ CH₃ oder COCH₃,
R² CH₃, 4,8,12-Trimethyl-tridecyl oder 4,8,12-Trimethyltrideka-3,7,11-trienyl und
X CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ oder CH(OCH₃)₂ oder eines der folgenden Strukturelemente X₁-X₇.

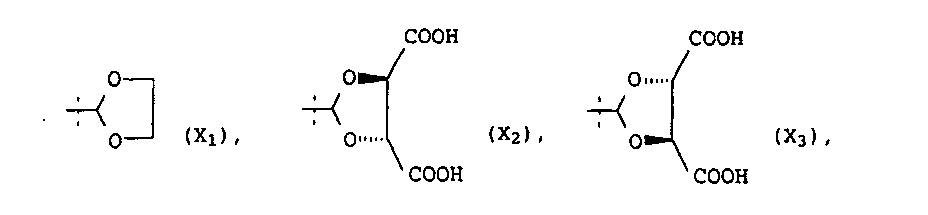

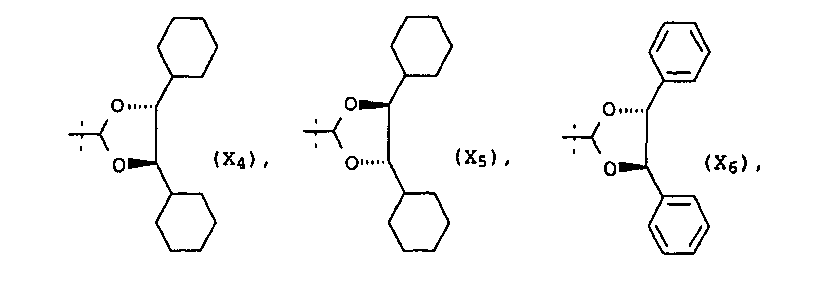

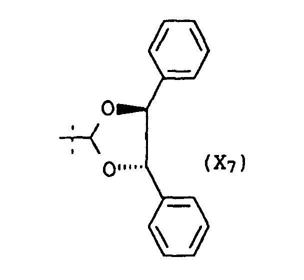

4. Verfahren zur Herstellung substituierter 3,4-Dihydro-2*H*-Pyrane der allgemeinen Formel III gemäß Anspruch 2, **dadurch gekennzeichnet, daß** man substituierte Acroleine der allgemeinen Formel I, 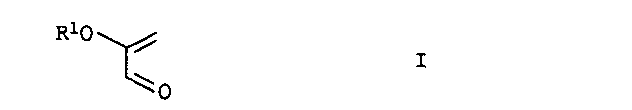 mit Acrylnitrilen, Acrylaten, Acroleinen, Acroleinacetalen, Allylalkoholen oder Allylethern der allgemeinen Formel II 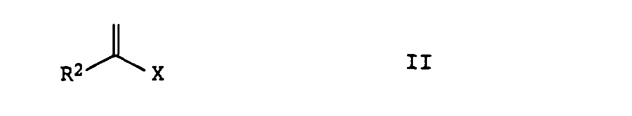 umsetzt.

5. Substituierte 5-Oxo-tetrahydropyrane der allgemeinen Formel IV, 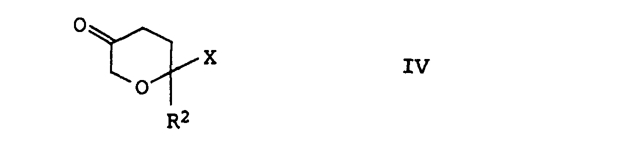 wobei
X die Gruppe -CN, -COOR³, -CHO, -CH₂OR⁷ oder -CH(OR⁸)₂,
R² einen C₁-C₂₃-Alkyl-, C₂-C₂₃-Alkenyl, C₆-C₁₈-Aryl-, oder C₇-C₁₈-Aralkyl-Rest,
R³ Wasserstoff, einen C₁-C₄-Alkylrest, einen C₁-C₄-Halogenalkylrest, einen C₁-C₄-Hydroxyalkylrest oder einen C₁-C₄-Aminoalkylrest,
R⁷ Wasserstoff oder einen C₁-C₄-Alkylrest
R⁸ einen C₁-C₄-Alkylrest, oder beide Reste einen C₂-C₆-Alkylenrest bedeuten, der die beiden Sauerstoffatome im Sinne eines cyclischen Acetals verbrückt und gegebenenfalls verzweigt ist oder ein bis zwei Carboxylgruppen, Cyclohexyl- oder Phenylreste tragen kann,
bedeuten.

6. Substituierte 5-Oxo-tetrahydropyrane der allgemeinen Formel IV nach Anspruch 5, wobei die Substituenten folgende Bedeutung haben:
R² CH₃, 4,8,12-Trimethyl-tridecyl oder 4,8,12-Trimethyltrideka-3,7,11-trienyl und
X CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ oder CH(OCH₃)₂ oder eines der folgenden Strukturelemente X₁-X₇.

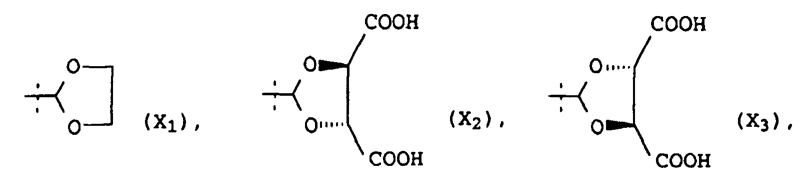

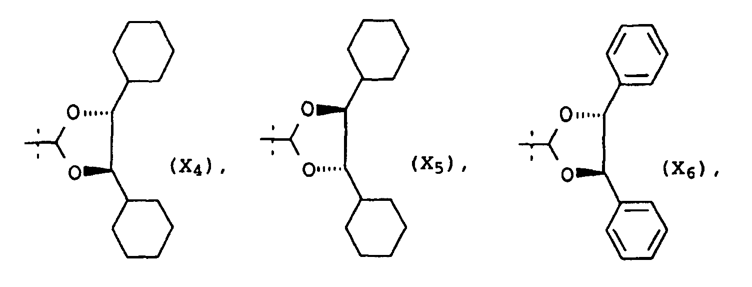

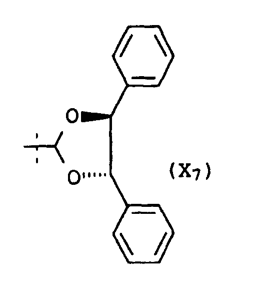

7. Verfahren zur Herstellung substituierter 5-Oxo-tetrahydropyrane der Formel IV gemäß Anspruch 6 **dadurch gekennzeichnet, daß** man substituierte Acroleine der allgemeinen Formel I, 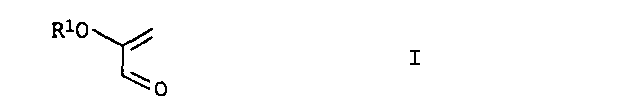 wobei
R¹ einen C₁-C₄-Alkyl-, C₆-C₁₈-Aryl-, C₇-C₁₈-Aralkyl-, C₁-C₄-Acyl-, einen halogenierten C₁-C₄-Acylrest oder eine andere säurelabile Schutzgruppe der Hydroxyfunktion darstellt,
mit Acrylnitrilen, Acrylaten, Acroleinen, Acroleinacetalen, Allylalkoholen oder Allylethern der allgemeinen Formel II 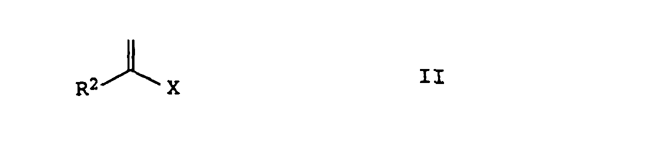 zu den entsprechenden 3,4-Dihydro-2*H*-Pyranen der allgemeinen Formel III 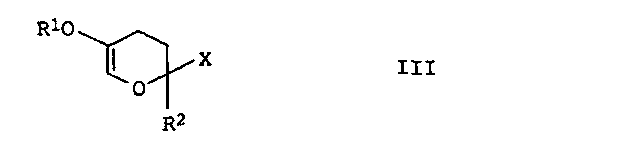 umsetzt und diese in einem zweiten Schritt mit einer Säure in die 5-Oxo-tetrahydropyrane der Formel IV überführt.

8. Substituierte Chromen-6-on-Derivate der allgemeinen Formel VI, 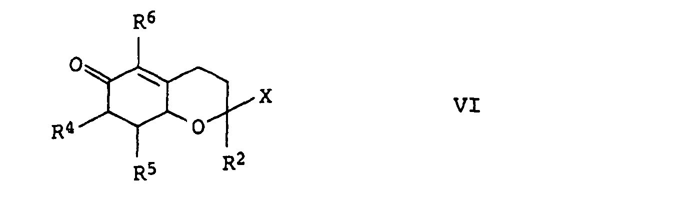 wobei
X die Gruppe -CN, -COOR³, -CHO, -CH₂OR⁷ oder -CH(OR⁸)₂,
R² einen C₁-C₂₃-Alkyl-, C₂-C₂₃-Alkenyl, C₆-C₁₈-Aryl-, oder C₇-C₁₈-Aralkyl-Rest,
R³ Wasserstoff, einen C₁-C₄-Alkylrest, einen C₁-C₄-Halogenalkylrest, einen C₁-C₄-Hydroxyalkylrest oder einen C₁-C₄-Aminoalkylrest,
R⁴, R⁵, R⁶ unabhängig voneinander Wasserstoff oder einen C₁-C₄-Alkylrest,
R⁷ Wasserstoff oder einen C₁-C₄-Alkylrest
R⁸ einen C₁-C₄-Alkylrest, oder beide Reste einen C₂-C₆-Alkylenrest bedeuten, der die beiden Sauerstoffatome im Sinne eines cyclischen Acetals verbrückt und gegebenenfalls verzweigt ist oder ein bis zwei Carboxylgruppen, Cyclohexyl- oder Phenylreste tragen kann,
bedeuten.

9. Substituierte Chromen-6-on-Derivate der allgemeinen Formel VI gemäß Anspruch 8, wobei die Substituenten folgende Bedeutung haben:
R² CH₃, 4,8,12-Trimethyl-tridecyl oder 4,8,12-Trimethyltrideka-3,7,11-trienyl,
R⁴, R⁵ und R⁶ unabhängig voneinander Wasserstoff oder CH₃ und
X CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ oder CH(OCH₃)₂ oder eines der folgenden Strukturelemente X₁-X₇.

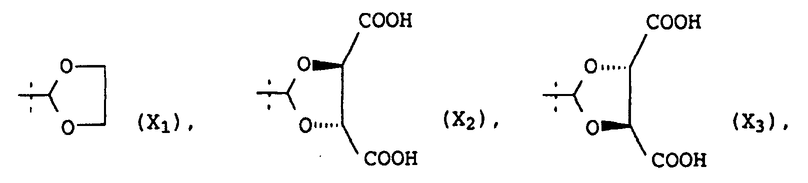

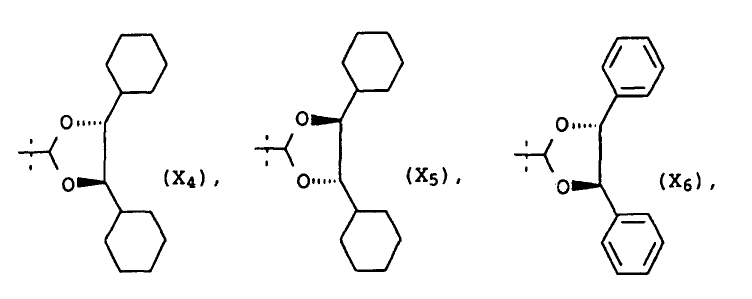

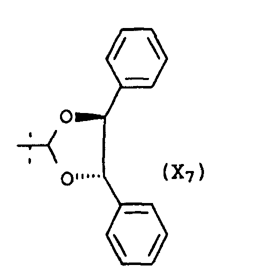

## Claims

1. A process for preparing substituted chroman derivatives of the general formula VII, 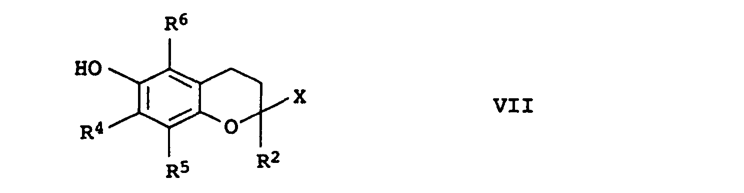 where
X is the group -CN, -COOR³, -CHO, -CH₂OR⁷ or -CH(OR⁸)₂,
R² is a C₁-C₂₃-alkyl, C₂-C₂₃-alkenyl, C₆-C₁₈-aryl or C₇-C₁₈-aralkyl radical,
R³ is hydrogen, a C₁-C₄-alkyl radical, a C₁-C₄-haloalkyl radical, a C₁-C₄-hydroxyalkyl radical or a C₁-C₄-aminoalkyl radical,
R⁴, R⁵, R⁶ are, independently of one another, hydrogen or a C₁-C₄-alkyl radical,
R⁷ is hydrogen or a C₁-C₄-alkyl radical,
R⁸ is a C₁-C₄-alkyl radical, or the two radicals are a C₂-C₆-alkylene radical which links the two oxygen atoms to form a cyclic acetal and is optionally branched or may carry one or two carboxyl groups, cyclohexyl or phenyl radicals,
which comprises, in a first step, reacting substituted acroleins of the general formula I, 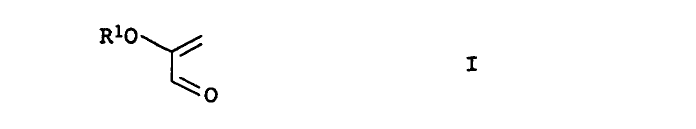 where
R¹ is a C₁-C₄-alkyl, C₆-C₁₈-aryl, C₇-C₁₈-aralkyl, C₁-C₄-acyl, a halogenated C₁-C₄-acyl radical or another acid-labile protective group for the hydroxyl group,
with acrylonitriles, acrylates, acroleins, acrolein acetals, allyl alcohols or allyl ethers of the general formula II 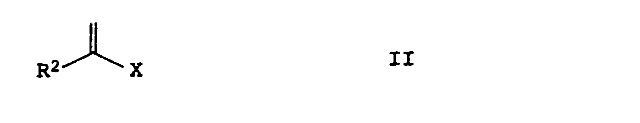 to give the corresponding 3,4-dihydro-2*H*-pyrans of the general formula III 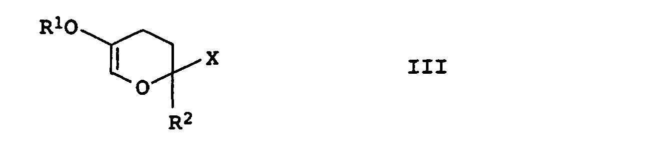 and, in a second step, reacting the latter with an acid to give the corresponding 5-oxotetrahydropyrans of the general formula IV 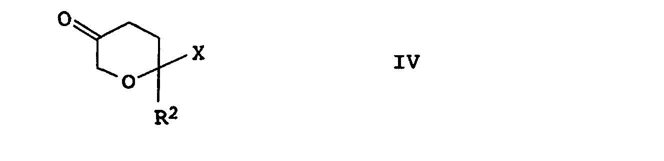 and, in a third step, reacting the latter with substituted vinyl ketones of the general formula V 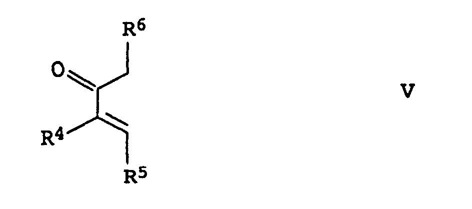 to give the corresponding chromen-6-one derivatives of the general formula VI 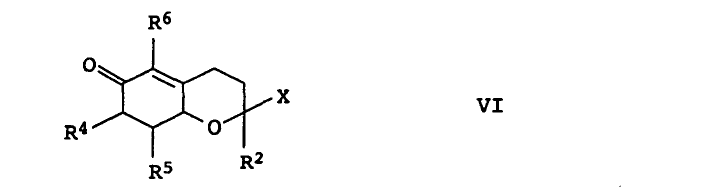 and, in a 4th step, dehydrogenating the latter to give the substituted chroman derivatives of the general formula VII.

2. A substituted 3,4-dihydro-2*H*-pyran of the formula III, 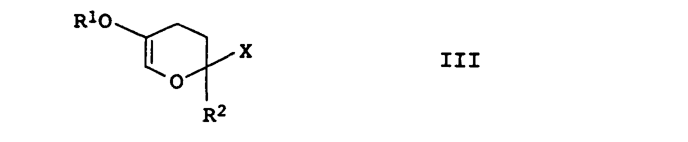 where
R¹ is a C₁-C₄-alkyl, C₆-C₁₈-aryl, C₇-C₁₈-aralkyl, C₁-C₄-acyl, a halogenated C₁-C₄-acyl radical or another acid-labile protective group for the hydroxyl group,
X is the group -CN, -COOR³, -CHO, -CH₂OR⁷ or -CH(OR⁸)₂,
R² is a C₁-C₂₃-alkyl, C₂-C₂₃-alkenyl, C₆-C₁₈-aryl or C₇-C₁₈-aralkyl radical,
R³ is hydrogen, a C₁-C₄-alkyl radical, a C₁-C₄-haloalkyl radical, a C₁-C₄-hydroxyalkyl radical or a C₁-C₄-aminoalkyl radical,
R⁷ is hydrogen or a C₁-C₄-alkyl radical,
R⁸ is a C₁-C₄-alkyl radical, or the two radicals are a C₂-C₆-alkylene radical which links the two oxygen atoms to form a cyclic acetal and is optionally branched or may carry one or two carboxyl groups, cyclohexyl or phenyl radicals.

3. A substituted 3,4-dihydro-2*H*-pyran of the formula III as claimed in claim 2, where the substituents have the following meanings:
R¹ CH₃ or COCH₃,
R² CH₃, 4,8,12-trimethyltridecyl or 4,8,12-trimethyl-3,7,11-tridecatrienyl and
X CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ or CH(OCH₃)₂ or one of the following structural elements X₁-X₇.

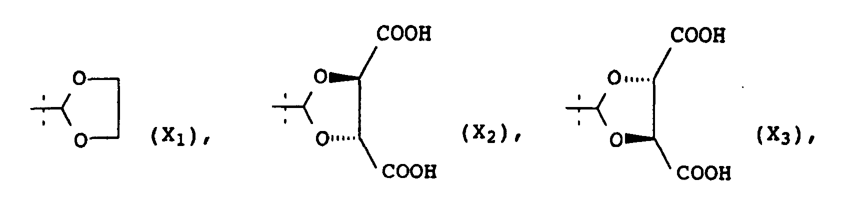

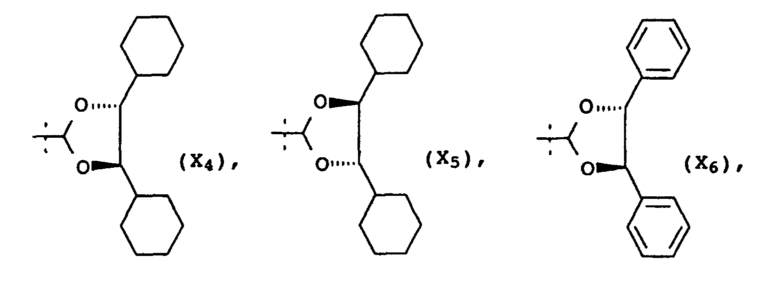

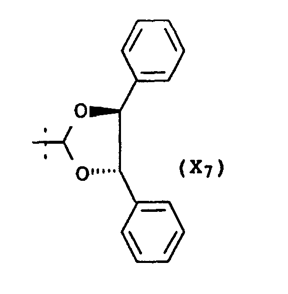

4. A process for preparing substituted 3,4-dihydro-2*H*-pyrans of the general formula III as claimed in claim 2, which comprises reacting substituted acroleins of the general formula I, 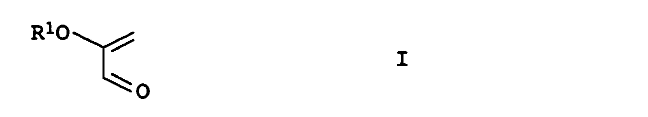 with acrylonitriles, acrylates, acroleins, acrolein acetals, allyl alcohols or allyl ethers of the general formula II

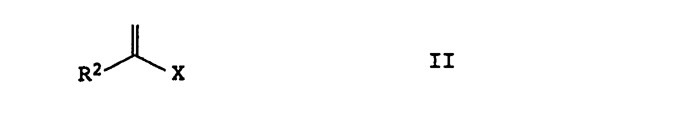

5. A substituted 5-oxotetrahydropyran of the formula IV, 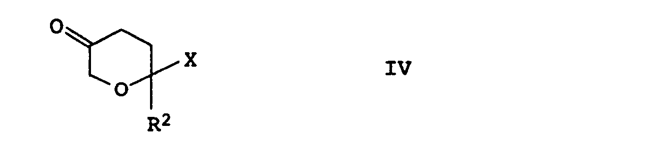 where
X is the group -CN, -COOR³, -CHO, -CH₂OR⁷ or -CH(OR⁸)₂,
R² is a C₁-C₂₃-alkyl, C₂-C₂₃-alkenyl, C₆-C₁₈-aryl or C₇-C₁₈-aralkyl radical,
R³ is hydrogen, a C₁-C₄-alkyl radical, a C₁-C₄-haloalkyl radical, a C₁-C₄-hydroxyalkyl radical or a C1-C4-aminoalkyl radical,
R⁷ is hydrogen or a C₁-C₄-alkyl radical,
R⁸ is a C₁-C₄-alkyl radical, or the two radicals are a C₂-C₆-alkylene radical which links the two oxygen atoms to form a cyclic acetal and is optionally branched or may carry one or two carboxyl groups, cyclohexyl or phenyl radicals.

6. A substituted 5-oxotetrahydropyran of the formula IV as claimed in claim 5, where the substituents have the following meanings:
R² CH₃, 4,8,12-trimethyltridecyl or 4,8,12-trimethyl-3,7,11-tridecatrienyl and
X CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ or CH(OCH₃)₂ or one of the following structural elements X₁-X₇.

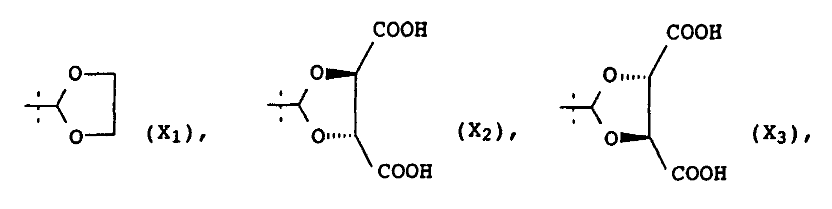

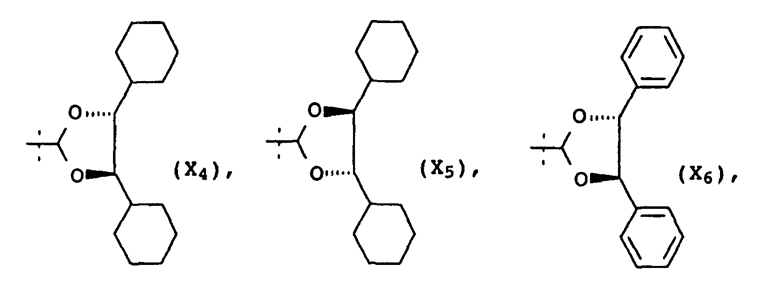

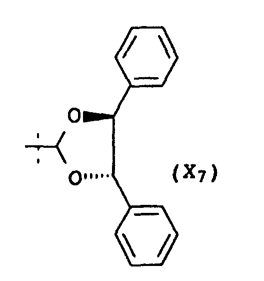

7. A process for preparing substituted 5-oxotetrahydropyrans of the formula IV as claimed in claim 6, which comprises reacting substituted acroleins of the general formula I, 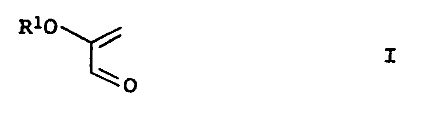 where
R¹ is a C₁-C₄-alkyl, C₆-C₁₈-aryl, C₇-C₁₈-aralkyl, C₁-C₄-acyl, a halogenated C₁-C₄-acyl radical or another acid-labile protective group for the hydroxyl group,
with acrylonitriles, acrylates, acroleins, acrolein acetals, allyl alcohols or allyl ethers of the general formula II 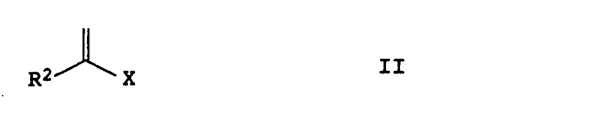 to give the corresponding 3,4-dihydro-2*H*-pyrans of the general formula III 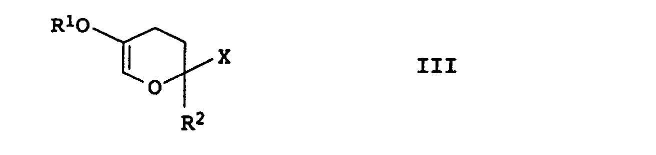 and, in a second step, converting the latter with an acid into the 5-oxotetrahydropyrans of the formula IV.

8. A substituted chromen-6-one derivative of the formula VI, 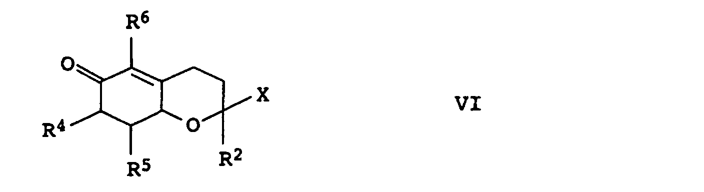 where
X is the group -CN, -COOR³, -CHO, -CH₂OR⁷ or -CH(OR⁸)₂,
R² is a C₁-C₂₃-alkyl, C₂-C₂₃-alkenyl, C₆-C₁₈-aryl or C₇-C₁₈-aralkyl radical,
R³ is hydrogen, a C₁-C₄-alkyl radical, a C₁-C₄-haloalkyl radical, a C₁-C₄-hydroxyalkyl radical or a C₁-C₄-aminoalkyl radical,
R⁴, R⁵, R⁶, independently of one another, are hydrogen or a C₁-C₄-alkyl radical,
R⁷ is hydrogen or a C₁-C₄-alkyl radical,
R⁸ is a C₁-C₄-alkyl radical, or the two radicals are a C₂-C₆-alkylene radical which links the two oxygen atoms to form a cyclic acetal and is optionally branched or may carry one or two carboxyl groups, cyclohexyl or phenyl radicals.

9. A substituted chromen-6-one derivative of the formula VI as claimed in claim 8, where the substituents have the following meanings:
R² CH₃, 4,8,12-trimethyltridecyl or 4,8,12-trimethyl-3,7,11-tridecatrienyl,
R⁴, R⁵ and R⁶ independently of one another hydrogen or CH₃ and
X CN, COOH, COOCH₃, COO-CH₂-CH₂-OH, COO-CH₂-CH₂-Cl, COO-CH₂-CH₂-N(CH₃)₂, CHO, CH₂OH, CH₂OCH₃ or CH(OCH₃)₂ or one of the following structural elements X₁-X_{7.}

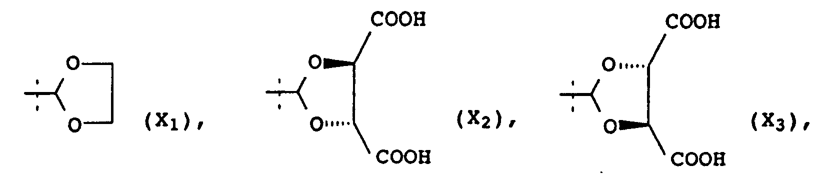

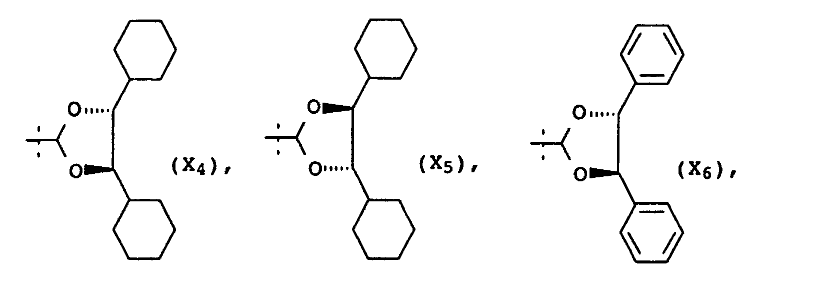

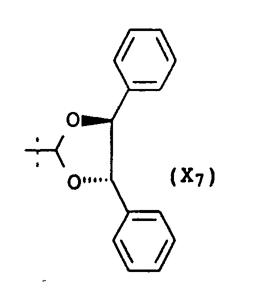

## Revendications

1. Procédé pour la préparation de dérivés de chromane substitués de formule générale VII, 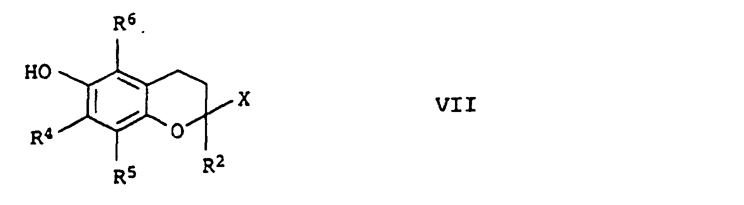 où
X désigne le groupe -CN, -COOR³, -CHO, -CH₂OR⁷ ou -CH(OR⁸)₂,
R² représente un reste alkyle en C₁-C₂₃, alcényle en C₂-C₂₃, aryle en C₆-C₁₈, ou aralkyle- en C₇-C₁₈,
R³ désigne l'hydrogène, un reste alkyle en C₁-C₄, un reste halogénoalkyle en C₁-C₄, un reste hydroxyalkyle en C₁-C₄ ou un reste aminoalkyle en C₁-C₄,
R⁴, R⁵, R⁶ désignent, indépendamment l'un de l'autre, l'hydrogène ou un reste alkyle en C₁-C₄,
R⁷ désigne l'hydrogène ou un reste alkyle en C₁-C₄,
R⁸ représente un reste alkyle en C₁-C₄, ou les deux restes forment un reste alkylène en C₂-C₆, qui ponte les deux atomes d'oxygène pour former un acétal cyclique, et qui est éventuellement ramifié ou peut porter un à deux restes carboxyle, cyclohexyle ou phényle,
**caractérisé par le fait qu'**on fait réagir dans une première étape des acroléines substituées de formule générale I, 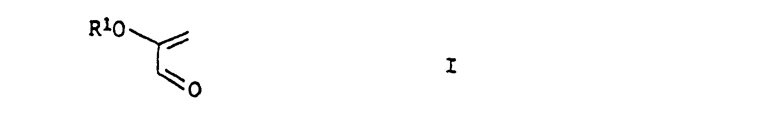 où
R¹ représente un reste alkyle en C₁-C₄, aryle en C₆-C₁₈, aralkyle en C₇-C₁₈, acyle en C₁-C₄, un reste acyle en C₁-C₄ halogéné ou un autre groupe protecteur de la fonction hydroxy, labile en présence d'acide,
avec des acrcylonitriles, des accrylates, des accroléines, des acétals d'acroléine, des alcools allyliques ou des éthers allyliques de formule générale II 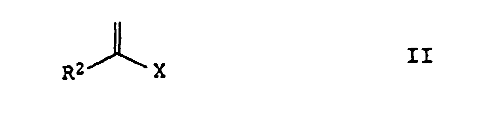 pour former les 3,4-dihydro-2*H*-pyrannes correspondants de formule générale III 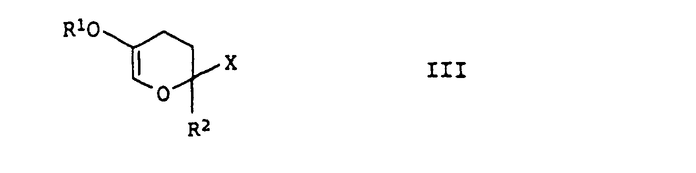 et on fait réagir ceux-ci dans une deuxième étape avec un acide pour former les 5-oxo-tétrahydropyrannes correspondants de formule générale IV 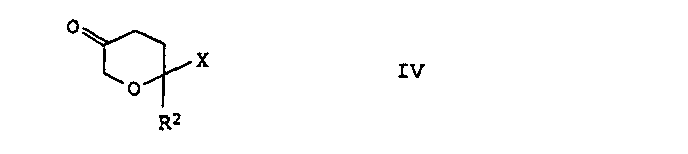 et on fait réagir ceux-ci dans une troisième étape avec des cétones vinyliques substituées de formule générale V 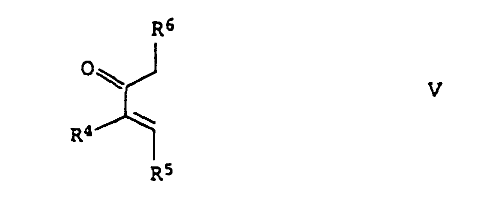 pour former les dérivés de chromène-6-one correspondants de formule générale VI 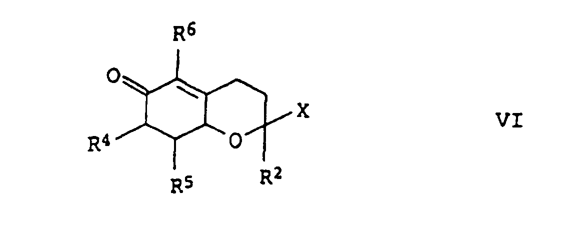 et on déshydrate ceux-ci dans une quatrième étape pour former les dérivés de chromane substitués de formule générale VII.

2. 3,4-dihydro-2*H*-pyrannes substitués de formule générale III, 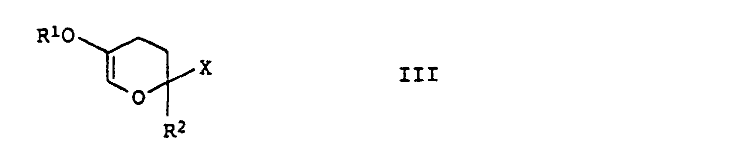 où
R¹ représente un reste alkyle en C₁-C₄, aryle en C₆-C₁₈, aralkyle en C₇-C₁₈, acyle en C₁-C₄, un reste acyle en C₁-C₄ halogéné ou un autre groupe protecteur de la fonction hydroxy, labile en présence d'acide,
X désigne le groupe -CN, -COOR³, -CHO, -CH₂OR⁷ ou -CH(OR⁸)₂,
R² représente un reste alkyle en C₁-C₂₃, alcényle en C₂-C₂₃, aryle en C₆-C₁₈, ou aralkyle en C₇-C₁₈,
R³ désigne l'hydrogène, un reste alkyle en C₁-C₄, un reste halogénoalkyle en C₁-C₄, un reste hydroxyalkyle en C₁-C₄ ou un reste aminoalkyle en C₁-C₄,
R⁷ désigne l'hydrogène ou un reste alkyle en C₁-C₄,
R⁸ représente un reste alkyle en C₁-C₄, ou les deux restes forment un reste alkylène en C₂-C₆, qui ponte les deux atomes d'oxygène pour former un acétal cyclique, et qui est éventuellement ramifié ou peut porter un à deux restes carboxyle, cyclohexyle ou phényle.

3. 3,4-dihydro-2*H*-pyrannes substitués de formule III selon la revendication 2, dans lesquels les substituants ont la signification suivante:
R¹ CH₃ ou COCH₃,
R² CH₃, 4,8,12-triméthyl-tridécyle ou 4,8,12-triméthyl-tridéca-3,7,11-triényle et
X CN, COOH, COOCH₃, COO-CH₂-CH₂-OH, COO-CH₂-H₂-Cl, COO-CH₂-CH₂-N(CH₃)₂, CHO, CH₂OH, CH₂OCH₃ ou CH(OCH₃)₂ ou l'un des éléments structuraux X₁-X₇ suivants

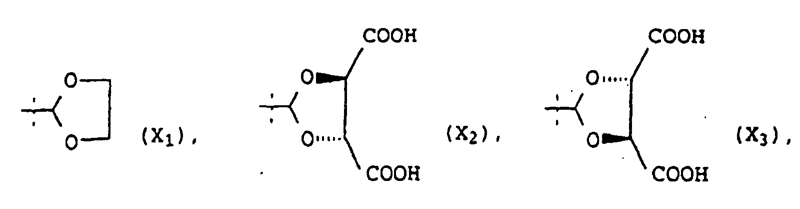

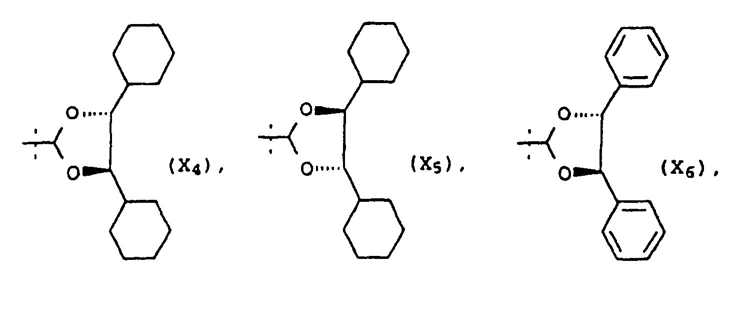

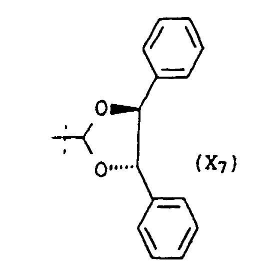

4. Procédé pour la préparation de 3,4-dihydro-2*H*-pyrannes substitués de formule générale III selon la revendication 2, **caractérisé par le fait qu'**on fait réagir des acroléines substituées de formule générale I, 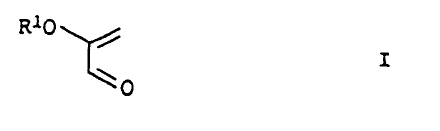 avec des acrylonitriles, des acrylates, des acroléines, des acétals d'acroléine, des alcools allyliques ou des éthers d'allyle de formule générale II

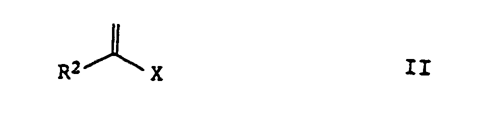

5. 5-oxo-tétrahydropyrannes substitués de formule générale IV, 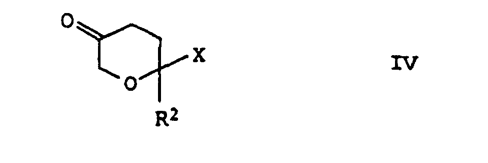 où
X désigne le groupe -CN, -COOR³, -CHO, -CH₂OR⁷ ou -CH(OR⁸)₂,
R² représente un reste alkyle en C₁-C₂₃, alcényle en C₂-C₂₃, aryle en C₆-C₁₈, ou aralkyle en C₇-C₁₈,
R³ désigne l'hydrogène, un reste alkyle en C₁-C₄, un reste halogénoalkyle en C₁-C₄, un reste hydroxyalkyle en C₁-C₄ ou un reste aminoalkyle en C₁-C₄,
R⁷ désigne l'hydrogène ou un reste alkyle en C₁-C₄,
R⁸ représente un reste alkyle en C₁-C₄, ou les deux restes forment un reste alkylène en C₂-C₆, qui ponte les deux atomes d'oxygène pour former un acétal cyclique, et qui est éventuellement ramifié ou peut porter un à deux restes carboxyle, cyclohexyle ou phényle.

6. 5-oxo-tétrahydropyrannes substitués de formule générale IV selon la revendication 5, dans lesquels les substituants ont la signification suivante:
R² CH₃, 4,8,12-triméthyl-tridécyle ou 4,8,12-triméthyl-tridéca-3,7,11-triényle et
X CN, COOH, COOCH₃, COO-CH₂-CH₂-OH, COO-CH₂-H₂-Cl, COO-CH₂-CH₂-N(CH₃)₂, CHO, CH₂OH, CH₂OCH₃ ou CH(OCH₃)₂ ou l'un des éléments structuraux X₁-X₇ suivants

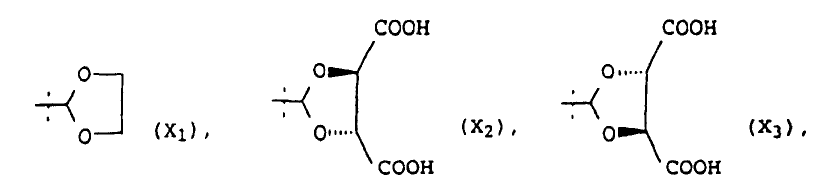

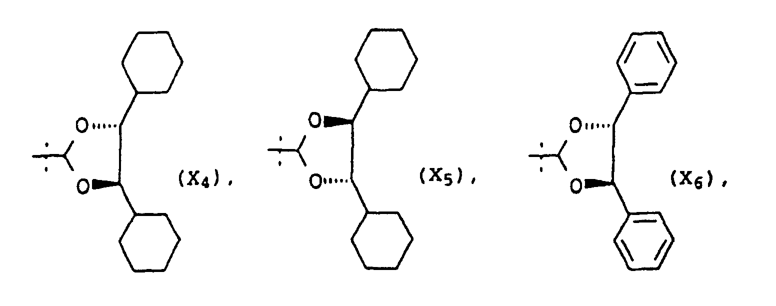

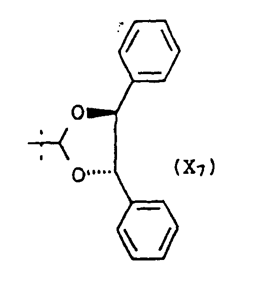

7. Procédé pour la préparation de 5-oxo-tétrahydropyrannes substitués de formule V selon la revendication 6, **caractérisé par le fait qu'**on fait réagir des acroléines substituées de formule générale I, 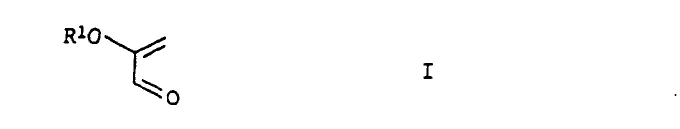 où
R¹ désigne un reste alkyle en C₁-C₄, aryle en C₆-C₁₈, aralkyle en C₇-C₁₈, acyle en C₁-C₄, acyle en C₁-C₄ halogéné, ou un autre groupe protecteur de la fonction hydroxy, labile en présence d'acide,
avec des acrylonitriles, des acrylates, des acroléines, des acétals d'acroléine, des alcools allyliques ou des éthers d'allyle de formule générale II 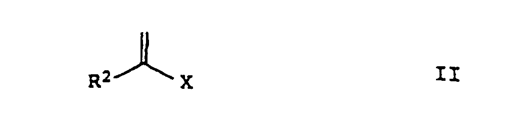 pour donner les 3,4-dihydro-2*H*-pyrannes correspondants de formule générale III 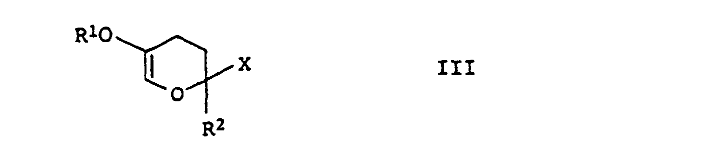 et dans une deuxième étape on transforme ceux-ci avec un acide en les 5-oxo-tétrahydropyrannes de formule IV.

8. Dérivés de chromène-6-one substitués de formule générale VI, 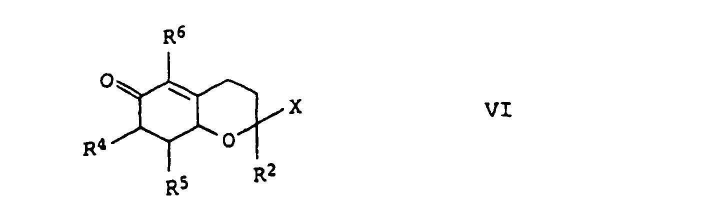 où
X désigne le groupe -CN, -COOR³, -CHO, -CH₂OR⁷ ou -CH(OR⁸)₂,
R² représente un reste alkyle en C₁-C₂₃, alcényle en C₂-C₂₃, aryle en C₆-C₁₈, ou aralkyle en C₇-C₁₈,
R³ désigne l'hydrogène, un reste alkyle en C₁-C₄, un reste halogénoalkyle en C₁-C₄, un reste hydroxyalkyle en C₁-C₄ ou un reste aminoalkyle en C₁-C₄,
R⁴, R⁵, R⁶ désignent, indépendamment l'un de l'autre, l'hydrogène ou un reste alkyle en C₁-C₄,
R⁷ désigne l'hydrogène ou un reste alkyle en C₁-C₄,
R⁸ représente un reste alkyle en C₁-C₄, ou les deux restes forment un reste alkylène en C₂-C₆, qui ponte les deux atomes d'oxygène pour former un acétal cyclique, et qui est éventuellement ramifié ou peut porter un à deux restes carboxyle, cyclohexyle ou phényle.

9. Dérivés de chromène-6-one substitués de formule générale VI selon la revendication 8, dans lesquels les substituants ont la signification suivante:
R² CH₃, 4,8,12-triméthyl-tridécyle ou 4,8,12-triméthyl-tridéca-3,7,11-triényle,
R⁴, R⁵ et R⁶ indépendamment l'un de l'autre hydrogène ou CH₃ et
X CN, COOH, COOCH₃, COO-CH₂-CH₂-OH, COO-CH₂-H₂-Cl, COO-CH₂-CH₂-N(CH₃)₂, CHO, CH₂OH, CH₂OCH₃ ou CH(OCH₃)₂ ou l'un des éléments structuraux X₁-X₇ suivants
